(19) Europäisches Patentamt

European Patent Office

Office européen des brevets

(11) **EP 1 033 959 B1**

(12) **EUROPEAN PATENT SPECIFICATION**

(45) Date of publication and mention
of the grant of the patent:
**27.08.2003 Bulletin 2003/35**

(51) Int Cl.[7]: **A61F 13/72**, A61F 13/15

(21) Application number: **97946938.4**

(86) International application number:
**PCT/US97/20791**

(22) Date of filing: **13.11.1997**

(87) International publication number:
**WO 99/025300 (27.05.1999 Gazette 1999/21)**

(54) **NON-UNITARY ABSORBENT ARTICLES**

AUS VERSCHIEDENEN TEILEN BESTEHENDE ABSORBIERENDE ARTIKEL

ARTICLES ABSORBANTS A PLUSIEURS PIECES

(84) Designated Contracting States:
**AT BE CH DE DK ES FI FR GB GR IE IT LI LU NL
PT SE**

(43) Date of publication of application:
**13.09.2000 Bulletin 2000/37**

(73) Proprietor: **THE PROCTER & GAMBLE COMPANY
Cincinnati, Ohio 45202 (US)**

(72) Inventors:
  • **WILLMS, Eric, Joachim
    D-65812 Bad Soden (DE)**
  • **SCHMITT, Achim
    D-55424 Munster-Sarmsheim (DE)**
  • **WOSCHNIK, Thomas
    D-53881 Euskirchen (DE)**
  • **MICCIO, Silvio
    D-53123 Bonn (DE)**
  • **THURNAY, Eva, Susanne
    D-76344 Eggenstein-Leopoldshafen (DE)**
  • **BLANCO, Ramos Augustin
    D-61440 Oberursel (DE)**

(74) Representative: **Hirsch, Uwe Thomas et al
Procter & Gamble European Service GmbH,
Sulzbacher Strasse 40-50
65824 Schwalbach am Taunus (DE)**

(56) References cited:
EP-A- 0 631 768          WO-A-93/01785
WO-A-96/41602          WO-A-97/04730
DE-C- 435 579           GB-A- 2 282 522
GB-A- 2 294 865         US-A- 2 052 598
US-A- 5 127 911         US-A- 5 454 802

**Description**

General field of the invention

The present invention relates to hygienic absorbent articles, in particular to absorbent articles designed for receiving larger amounts of body fluids as urine and / or fecal material, such as adult incontinence articles or baby diapers, and more particular to non-unitary articles, such as articles comprising a separate absorbent element and a fixation element such as a garment.

Background / Prior art

Absorbent articles for use in hygienic applications are well known in the art. Such articles always at least comprise an absorbent member to receive and retain the bodily exudates, and fixation means to hold the absorbent member in registration with the body openings which release the respective body exudates.

One class of articles well known in the art are "unitary" articles, i.e. the absorbent member and the fixation means form essentially one product. Such articles are widely used in the field of baby diapers, with typical tape constructions serving as a fixation means by allowing the article to be wrapped around the body of the wearer thereby being fixed on the body during use. Other unitary articles are diapers of the pant type, or absorbent pads which are affixed directly to the underwear of the wearer, as such as well known for so called "panty-liner" or other pads in the feminine hygiene or light incontinence field.

Another class of articles are non-unitary ones, whereby the absorbent element and the fixation element can be separated. Examples are so-called "pad-pant" systems, such as well known in the adult incontinence field.

For all systems, there have been attempts to improve the system, and in particular to improve the sustained fit of the pad, i.e. holding it in its position even under longer wearing times and loadings.

These attempts have been made on the absorbent element, or on the fixation element.

Improved absorbent elements have been disclosed e.g. in US-A-5.300.055 (Buell) with an absorbent article having a body facing surface which has a convex upward surface, such that the total article either takes the shape of an "inverted U" (i.e. the opening of the U facing away from the wearer) or of a "W". In WO 95/31165 (Olsen) a similar article is described, aiming at a "W" shaped configuration by means of a resilient component as an integral part of the article.

In WO 95/17148 (Bergman), sanitary napkins are disclosed, which achieve an improved contact between the labia of the wearer and the article for improved fluid (i.e. menses) handling capability. Also WO 88/04547 (Thoren) describes a sanitary napkin aiming at improved contact between the article and the wearer by attaching elastic members both in MD and in CD direction to that side of the topsheet which is oriented away from the wearer. Also EP-A-0.302.523 (Lassen) describes an anatomically - shaped feminine pad, made by forming, molding or other forming techniques.

Other approaches aimed at improving the fixation system.

Prior art developments includes GB 2 282 053, which describes a panty that may be used by men who need to wear an absorbent product in the region of their groin as a result of a medical condition. The panty is so configured and constructed that, in use, the absorbent product firmly embraces the body of the wearer by the panty via a partial lining of impermeable material, and movement of t the absorbent product is prevented. This solution has a degree of effect, but problems still arise with regard to tightness which may cause wearer discomfort, rolling up of the leg areas leading to leakage of fluids, and wearer movement can create gapping in the groin and back regions.

GB 2 185 678 A discloses a disposable undergarment comprising an integral absorbent pad that can function as a light incontinence garment. The absorbent pad stretches upwards from the crotch region both in the back and the front region to a point higher than normal absorbent pads. The device is designed to substantially minimize the leakage of fluids in overnight use. The configuration may lead to wearer discomfort due to the built-in and high positioning of the absorbent pad.

US 4,355,425 describes an improved panty and method of making the same that has both nonwoven porous fabric panels and nonwoven elastic members. The panty is characterized by elastication in all directions. Problems may arise regarding body contact and the positioning of the absorbent product. Furthermore, the panty is only designed from use by a woman or a child.

WO 92/00051 discloses an undergarment that includes a permanently stretched region within which the incontinence guard is placed and in which the material has a lower elasticity than tin the remaining regions of the undergarment. Such features enable the correct and ready positioning of the incontinence guard and improved wearer confidence. Nevertheless, the configuration leads to bunching in the back region and does not guarantee a high degree of body contact. Furthermore, when the incontinence guard is loaded, the undergarment is incapable of covering the incontinence guard effectively and leakage may occur.

WO 95/09594 relates to a light incontinence panty that is characterized by elastic devices, which extend from the front to the back part of the panty. The elastic devices may comprise elastic threads, ribbons or bands that are preferably mounted between two layers or sheets comprising the panty. The invention however does not disclose a garment that incorporates elasticity through an integral knitting technology. The panty may suffer from such drawbacks as wearer discomfort, sagging of the waistband and a poor fit.

U.S. Patent 5,611,722, issued to Osborn on March 18, 1997 describes a panty-type undergarment. The panty-type undergarment has a front panel, a rear panel, and a crotch portion. The undergarment further includes a substantially anchor-shaped support panel having a greater resistance to stretch than the rest of the undergarment which is integrally knit into the rear panel. The support panel is said to lift and separate the cheeks of a wearer's buttocks. The support panel includes a vertical strip and upwardly curving portions which extend toward and along a portion of the undergarment's leg openings. While such undergarments may lift and separate the cheeks of a wearer's buttocks, the undergarments fail to provide a lifting force that would improve bodily contact between a catamenial device and a wearer's pudendal region.

GB 2, 282, 522 discloses an absorbent article comprising an absorbent element comprising a unitary loading zone and a garment for holding said absorbent element.

However, the prior art failed to recognize the particular benefits, which arise from combining an absorbent element and a fixation element in the particular way of the present invention.

Objects of the invention

Hence it is an object of the present invention to provide non-unitary absorbent articles particularly designed for receiving relatively large amounts of body exudates such as urine or feces, whereby the articles do not raise the discomfort for the wearer unduly, without detrimentally impacting on performance of the article.

It is a further aspect of the present invention, to provide such articles having additionally improved handling capability with regard to feces.

It is a further object of the present invention to provide designs which at the same time satisfy these requirements and allow easy application to the wearer, either by him- or herself, or by another person such as a parent or a caretaker.

The present invention relates to absorbent articles comprising an absorbent element and a fixation garment so as to achieve or maintain a convexo-concave shape of the absorbent core in the absorbent element with an upwardly bulged (i.e. towards the wearer during use) center crotch part, and outwardly bulged parts at the longitudinal ends of the absorbent element following the body curvature to surround the waist.

Brief description of drawings

Figure 1 shows an absorbent pad as an example of an absorbent element;

Figure 2a shows articles bunching in the $\Omega$-shape according to the present invention;

Figure 2b shows a comparative prior art article in a W-fold shape;

Figure 3 is a front view of a preferred embodiment of the garment of the present invention.

Figure 4 is a rear view of the garment shown in Figure 1.

Figure 5 is a plan view of the garment shown in Figure 1 that has been opened at the sides, the elastic components being pulled flat.

Figure 6 is a rear view of an alternative embodiment of a garment of the present invention.

Figure 7 is a side view of an alternative embodiment of a garment of the present invention.

Figure 8 is a front view of the garment shown in Figure 7.

Figure 9 is a front view of an alternative embodiment of a garment of the present invention.

Detailed description

An absorbent article generally comprises:

- an absorbent member (often referred to as core or core structure, which may consist of sub-structures);
- "chassis elements", such as
- a fluid impervious backsheet;
- optionally further features like closure elements
- or elastification.

Within the context of the present description, an absorbent article can be "unitary", i.e. all elements are adjoined together such that the article is - when being used - essentially one piece, or the absorbent article can consist of separate elements, such as having an absorbent element such as an absorbent pad, and an external fixation element, e.g. a re-usable fixation stretch garment, such as often practiced for the adult incontinence articles in form of a elasticized pant combination with an absorbent disposable pad.

In either case, the article or its elements can be disposable or reusable, whereby the term "disposable" is used herein to describe absorbent articles or elements which are not intended to be laundered or otherwise restored or reused as an absorbent article or element (i.e. they are intended to be discarded after a single use and, preferably, to be recycled, composted or otherwise disposed of in an environmentally compatible manner).

The absorbent article has a lateral direction (x direction or width) defined as the direction parallel to the lateral centerline and being aligned with a "left-to-right" direction of the wearer when being used; the longitudinal direction (y direction or length) being defined as the direction parallel to the longitudinal centerline and being aligned with the height direction of a wearer in a standing position during use; and the axial direction (Z direction or thickness) being defined as the direction extending through the thickness of the diaper 20.

The term "concave" means - in the context of the present invention - a curvature which is "outwardly bulged", such as when following the general curvature of the waist of a human wearer, i.e. a belt worn around the waist would exhibit a generally concave curvature. The term "convex" relates to the opposite curvature. Of course, these terms are relative, and also linked to terms "upwards" and "downwards", which follow the general understanding of gravity. Henceforth, a letter "U" would has a "downward convex", or a concave, shape. The Greek capital letter Ω (Omega) has in the center part an "upwardly convex" curvature. If the terms "concave" and "convex" are used without an "upward' or "downward" direction, the meanings is as with "upward". If these terms are used in referring to a wearers position, this is assumed, unless otherwise noted, that the wearer is in a standing position.

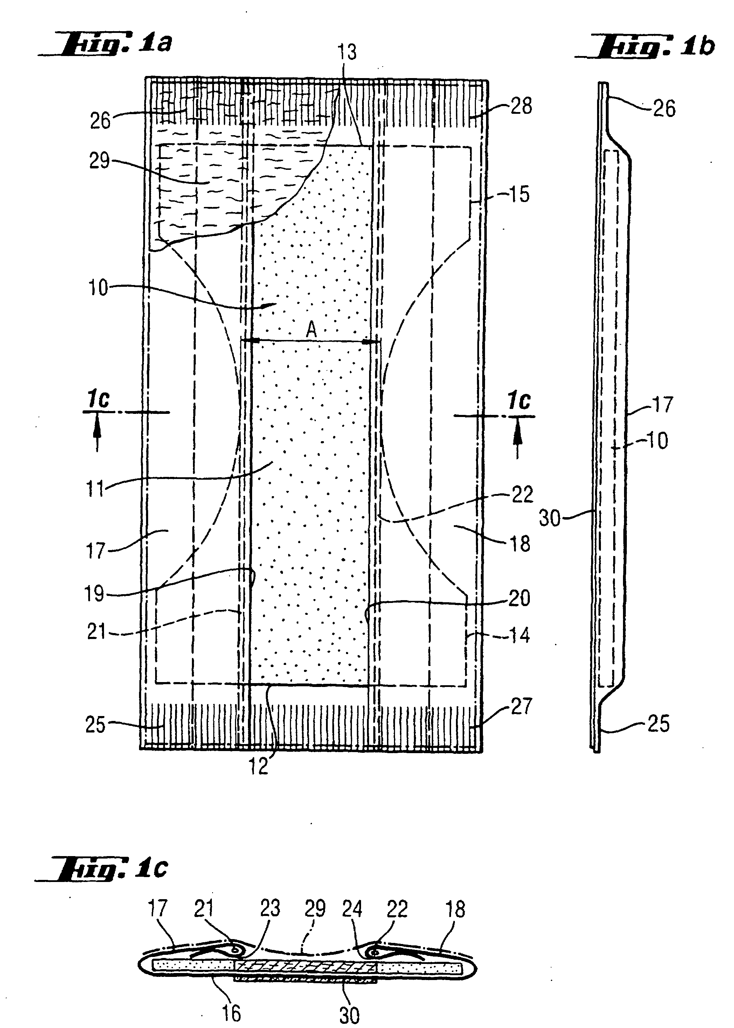

In Fig. 1 a, b, and c the present invention is exemplified by describing the absorbent element of an absorbent article, which could be a baby diaper or an adult incontinence pad.

The pad comprises an absorbent core 10, designed for absorbing and containing fluids, in most instances primarily aqueous based. The absorbent core 10 may be any absorbent means which is generally compressible, conformable, and capable of absorbing and retaining primarily aqueous liquids. Examples of suitable absorbent materials include comminuted wood pulp, creped cellulose wadding; meltblown polymers; chemically stiffened, modified or cross-linked cellulosic fibres; tissue including tissue wraps and tissue laminates; absorbent foams; absorbent sponges; superabsorbent polymers; absorbent gelling materials; or any equivalent material or combinations of materials. The configuration and construction of the absorbent pad may also be varied (e.g., the absorbent pad may have varying caliper zones, a hydrophilicity gradient, a superabsorbent gradient, or lower average density and lower average basis weight acquisition zones; or may comprise one or more layers or sub-structures). Preferably the absorbent pad has an essentially flat configuration so as to avoid complications which could arise from having a strongly threedimensionally shaped form.

Exemplary absorbent structures for use in the absorbent pad as used in the disposable industry are described in U.S. Patent 4,610,678 entitled "High-Density Absorbent Structures" issued to Weisman et al. on September 9, 1986; U.S. Patent 4,673,402 entitled "Absorbent Articles With Dual-Layered Cores" issued to Weisman et al. on June 16, 1987; U.S. Patent 4,888,231 entitled "Absorbent Core Having A Dusting Layer" issued to Angstadt on December 19, 1989; and U.S. Patent 4,834,735, entitled "High Density Absorbent Members Having Lower Density and Lower Basis Weight Acquisition Zones", issued to Alemany et al. on May 30, 1989. Other absorbent pad designs are described in European Patent Application No.'s EP 631 768 and EP 640 330.

US patent no 4,411,660 discloses in an absorbent product two layers of absorbent material of different types, such that the upper layer gels slower than the first layer.

European Patent Specification EP-B-0 401 189 discloses that favorable properties of absorbent products can be achieved by using two different types of absorbent gelling material in separate layers, rather than as a mixture of the two absorbent gelling materials in a single layer.

The hydrogel-forming absorbent polymers useful in the present invention include a variety of substantially water-insoluble, but water-swellable polymers capable of absorbing large quantities of liquids. Such polymers materials are also commonly referred to as "hydrocolloids", or "superabsorbent" materials. These hydrogel-forming absorbent polymers preferably have a multiplicity of anionic, functional groups, such as sulfonic acid, and more typically carboxy, groups. Examples of polymers suitable for use herein include those which are prepared from polymerizable, unsaturated, acid-containing monomers. Thus, such monomers include the olefinically unsaturated acids and anhydrides that contain at least one carbon to carbon olefinic double bond. More specifically, these monomers can be selected from olefinically unsaturated carboxylic acids and acid anhydrides, olefinically unsaturated sulfonic acids, and mixtures thereof.

As described above, the hydrogel-forming absorbent polymers are preferably slightly network crosslinked. Network crosslinking serves to render the polymer substantially water-insoluble and, in part, determines the absorptive capacity and extractable polymer content characteristics of the precursor particies and the resultant macrostructures. Processes for network crossiinking the polymers and typical network crossiinking agents are described in greater detail in the herein before-referenced U.S. Patent 4,076,663, and in DE-A-4020780 (Dahmen).

In order to be able to compare absorbent articles for varying end use conditions, or differently sized articles, the "design capacity" has been found to be a suitable measure.

For example, babies are representing a typical usage group, but even within this group the amount of urine loading, frequency of loading, composition of the urine will vary widely from smaller babies (new-born babies) to toddlers on one side, but also for example among various individual toddlers.

Another user group may be larger children, still suffering from a certain form of incontinence.

Also, incontinent adults can use such articles, again with a wide range of loading conditions, generally referred to as light incontinence ranging up to severe incontinence.

Henceforth, absorbent articles being able to cope with such requirements should have the capability of picking up such amounts of urine, which will be referred to for the further discussion as "design capacity", which is descibed in more detail below.

These amounts of fluids have to be absorbed by materials which can ultimately store the bodily fluids, or at least the aqueous parts of these, such that - if any - only little fluid is left on the surface of the article towards the wearers skin. The term "ultimate" refers in one respect to the situation as in the absorbent article at long wearing times, in the other respect to absorbent materials which reach their "ultimate" capacity when being equilibrated with their environment. This can be in such an absorbent article under real in-use conditions after long wearing times, or this also can be in a test procedure for pure materials or material composites. As many of the processes under consideration have asymptotic kinetic behavior, one skilled in the art will readily consider "ultimate" capacities to be reached when the actual capacity has reached a value sufficiently close to the asymptotic endpoint, e.g. relative to the equipment measurement accuracy.

As an absorbent article can comprise materials which are primarily designed to ultimately store fluids, and other materials which are primarily designed to fulfill other functions such as acquisition and/or distribution of the fluid, but may still have a certain ultimate storage capability, suitable core materials according to the present invention are described without attempting to artificially separate such functions. Nonetheless, the ultimate storage capacity can be determined for the total absorbent core, for regions thereof, for absorbent structures, or even sub-structures, but also for materials as being used in any of the previous.

As discussed in the above for varying the dimensions of the article, one skilled in the art will be able to readily adopt the appropriate design capacities for other intended user groups. For example, for Adult Incontinence articles intended for use with severely incontinent persons can contain 9 g of superabsorbent material having an absorbent capacity of about 31 ml/g when submitted to the well known Teabag centrifuge capacity test, and contain 97 g of conventional cellulosic airfelt having a capacity of about 4 ml/g, thus resulting in a total ultimate storage capacity of about 667 ml. Other examples relate to articles for light incontinent persons. For example ATTENDS MINI has an ultimate storage capacity of about 70 ml, ATTENDS MINI PLUS of 90 ml, or ATTENDS NORMAL of about 167 ml, with all these products sold by Procter & Gamble in various countries in Europe.

Referring again to Figures 1, said absorbent core has waist regions 14, 15 next to the longitudinal edges 12, 13, and a crotch region 11 connecting these regions 14, 15, which has a minimum width ÒA". As exemplified in Fig. 1a, the width in the waist region 14, 15 is about twice the width of the crotch region 11.

The absorbent core 10 is connected with a fluid impervious backsheet 16. The backsheet 16 may comprise a woven or nonwoven material, polymeric films such as thermoplastic films of polyethylene or polypropylene, or composite materials such as a film-coated nonwoven material. Often, the backsheet is a thermoplastic film having a thickness of from about 0.012 mm (0.5 mil) to about 0.051 mm (2.0 mils), such as a blown or cast PE film as available under the designation RR8220 (blown films) and RR5475 (cast films) as manufactured by Tredegar Industries, Inc. of Terre Haute, IN. Such a backsheet 16 is preferably embossed and/or matte finished to provide a more clothlike appearance.

Further, and often preferably, the backsheet 16 may permit vapours to pass through while still preventing liquids from penetrating through the backsheet 16.

Backsheet 16 may have both width and length dimensions exceeding the dimensions of the absorbent core 10, thereby forming peripheral edges. The region of the backsheet 16 which extend outwardly of the core in longitudinal direction is referred to as endflaps.

The relatively wide lateral edges 17, 18 of the backsheet 16 are folded inwardly so as to at least partially overlaying the absorbent core 10. The free longitudinal edges 19, 20 comprise an elastification feature 21, 22, such as an elastic band or stripe. When the absorbent article is stretched out flat as indicated in Fig. 1 these elastic features are stretched so as to allow contraction for better body conformity during use.

The lateral edges 17, 18 are now in a tubular arrangement 23, 24 with the unfolded parts, whereby the stretched elastics which are connected at their ends with the edges 25, 26 respectively 27, 28 of the inwardly folded lateral edges 17, 18.

A topsheet 29 is positioned on the absorbent core 10 oriented towards the wearer during use. The topsheet 29 is compliant, soft feeling. Further, the topsheet 29 is liquid pervious permitting fluids like urine liquids to readily penetrate through its thickness. A suitable topsheet may be manufactured from a wide range of materials, such as porous foams; reticulated foams; apertured plastic films; or woven or nonwoven webs of natural fibres (e.g., wood or cotton fibres), synthetic fibres (e.g., polyester or polypropylene fibres), or a combination of natural and synthetic fibres. There are a number of manufacturing techniques which may be used to manufacture the topsheet 29. For example, the topsheet 29 may be a nonwoven web of fibres spunbonded, carded, wet-laid, meltblown, hydroentangled, combinations of the above, or the like. An often used topsheet is carded and thermally bonded by means well known to those skilled in the fabrics art, for example made of staple length polypropylene fibres such as is manufactured by Veratec, Inc., a Division of International Paper Company, of Walpole, Massachusetts under the designation P-8.

The topsheet may be essentially attached throughout most or all of the surface of the absorbent core, or it may be only partially bonded thereto. The topsheet may comprise apertures to ease penetration of exudates there through, such as urine or feaces. The topsheet may further be fully or partially elasticated.

In order to keep the absorbent element as described in the above on the body of the wearer and in registry with the body fluid releasing body openings, the absorbent article has to have one or more fixation means.

Such a fixation means can of the mechanical type, i.e. essentially aiming at overcoming the gravitational forces and / or the frictional forces between the article and outside elements, such as the outer clothing of the wearer and /or the bedding elements by exploiting the frictional forces between the article and the skin of the wearer, or structural support forces such as registry with body contours such as hip bones of the wearer.

The absorbent element may alternatively be hold in place by conventional closure systems to fix the article or the absorbent element around the waist of the wearer, such as by integral tape systems, which can comprise adhesive coated tapes or mechanically engaging elements.

Thereby, the absorbent article or the absorbent element is provided with a closure system comprising a tape tab fastening device and a landing member. The tape tab fastening device comprises an attachment area and a functional area, and the landing member is preferably a reinforcing strip or the alternative a portion of the backsheet. The functional elements may take on a number of configurations such as adhesive fastening elements, mechanical fastening elements, a combination of adhesive fastening elements and mechanical fastening elements, or any other means as are known to the man skilled in the art.

For these closure systems which encircle the waist of the wearer, the article is preferably further comprising elastification features operatively connected with these closure feature, so as to enhance and sustain the fit of the article during use.

Absorbent articles, which are sometimes referred to as "pant-style diapers" are unitary articles with a pant style design - i.e. with closed side seams to form a fully closed structure in the waist region - together with elastification features to allow fixation as the mentioned elastic pant, and also an absorbent core such as a panty comprising elasticized region and further described in EP application number 811 362.

Alternatively, an absorbent element such as described in the above, may simply have a means for attaching it to the normal underwear of the user, such as often applied for light feminine hygiene articles, such as so-called "panty liner", and indicated in Fig. 1c by fixation means 30.

An even further alternative has a fastening means of the topical adhesive attachment type. Such "body adhesive" means aim at fixation of the article directly to the skin of the wearer, and are described such as in EP application number 9710666.91.

A further embodiment of the present invention relates to non-unitary articles, comprising an absorbent element and a separate fixation means or garment such as a net or stretch pant, which can be used either as replacement or in addition to the regular underwear of the wearer. A similar approach to such non-unitary elements can be an attachable belt as described in EP-A-0.409.307 (Gipson et al.)

Further, absorbent articles according to the present invention can have several of the above mentioned fixation means, such as having both a body adhesive applied to the absorbent element, and an additional stretch or garment in a dual fixation means arrangement.

In case of a non-unitary arrangement, both the absorbent element and the fixation element can be disposable, or one of these or both can be reusable, such as after washing.

Ω- (Omega)-shaped absorbent articles

In addition to the features as described above, the following embodiments make the present invention particularly suited for being used to receive urinary and / or fecal exudates.

The first and essential element is a feature to provide a convex bulging at least in the crotch region, and in particular at least in the urine loading region or zone, during application of the article to the wearer and / or during use. The urine loading zone is generally located somewhat, i.e. often about 5 cm, forward from the crotch point of the article, which corresponds to the narrowest width dimension of the stiffest part of the article.

This convex bulging can be achieved by means arranged in the crotch region of the article and in particular in the urinary loading zone of the article, with positioning the center line of the article during use in closer proximity to the body of the wearer than the lateral side edges of the absorbent member closer together than in a flat shape and thus have its center line positioned closer towards the wearer $\overline{O}$s body than the side edges, such that the absorbent member is in fact upwardly bulging towards the wearer.

In Fig. 2a, such an absorbent core 228 is forming the upwardly convex shape (i.e. the center part of an Ω (Omega). The lower parts of the Ω (Omega) are formed by chassis elements, i.e. primarily backsheet materials such as films and/or nonwovens. This configuration is referred to as "Ω-bunching".

This convex Ω-shape of the absorbent core has to be at least around the urinary loading point, preferably extending at least about +/- 2.5 cm to the front and towards the back of the article. In order to allow comfortable wearing, this convex shaping of the crotch region core will transition into a concavely shaped curvature of the front and rear waist parts of the article to fit well around the waist of the wearer.

The elastic elements to keep the side margins of the article in contact with the wearers legs are also positioned with the zones of the lower part of the Ω.

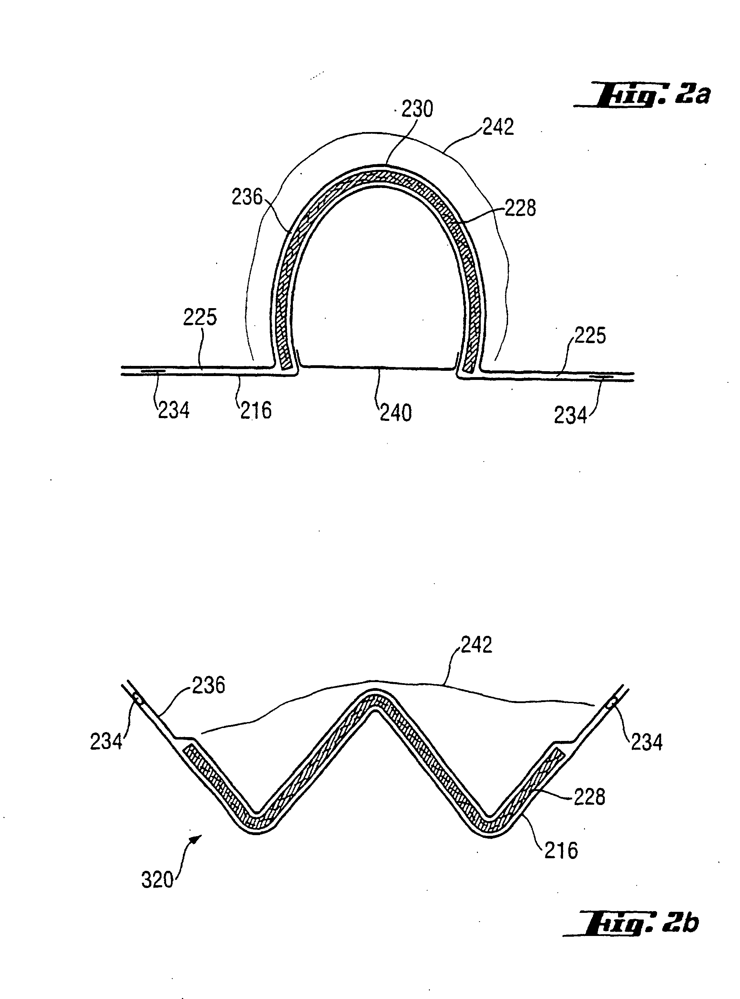

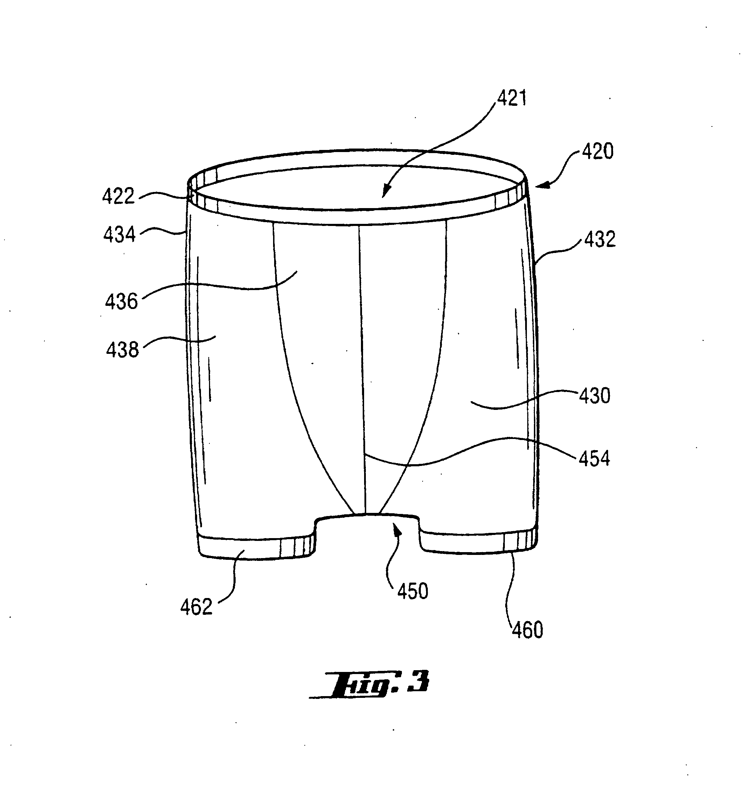

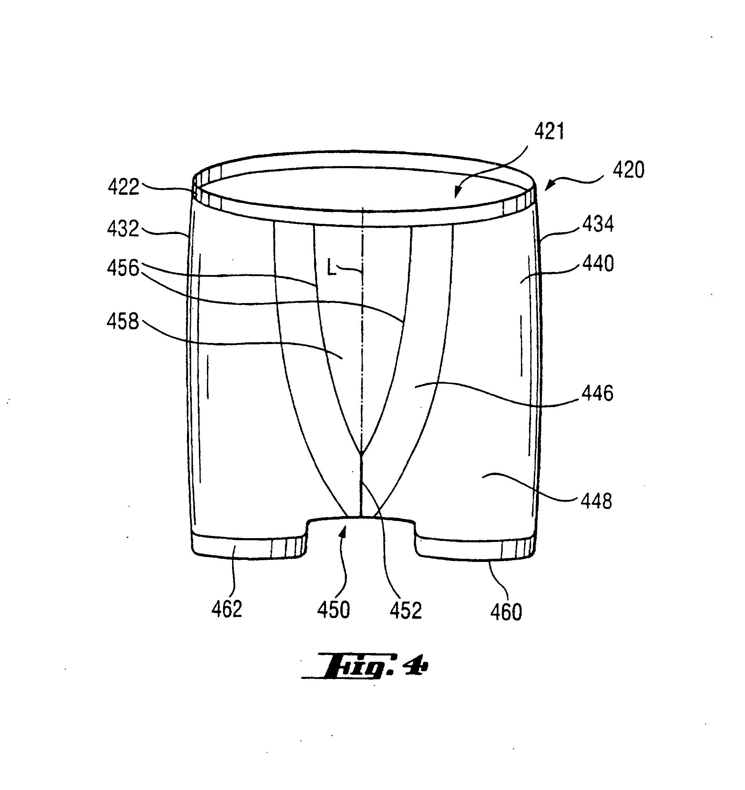

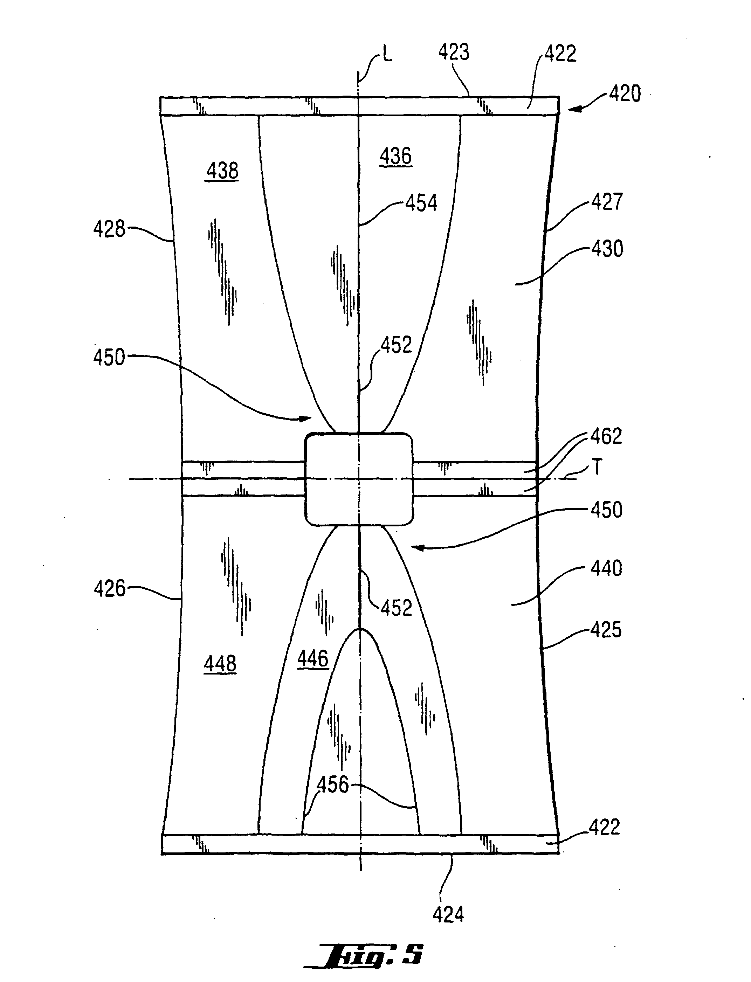

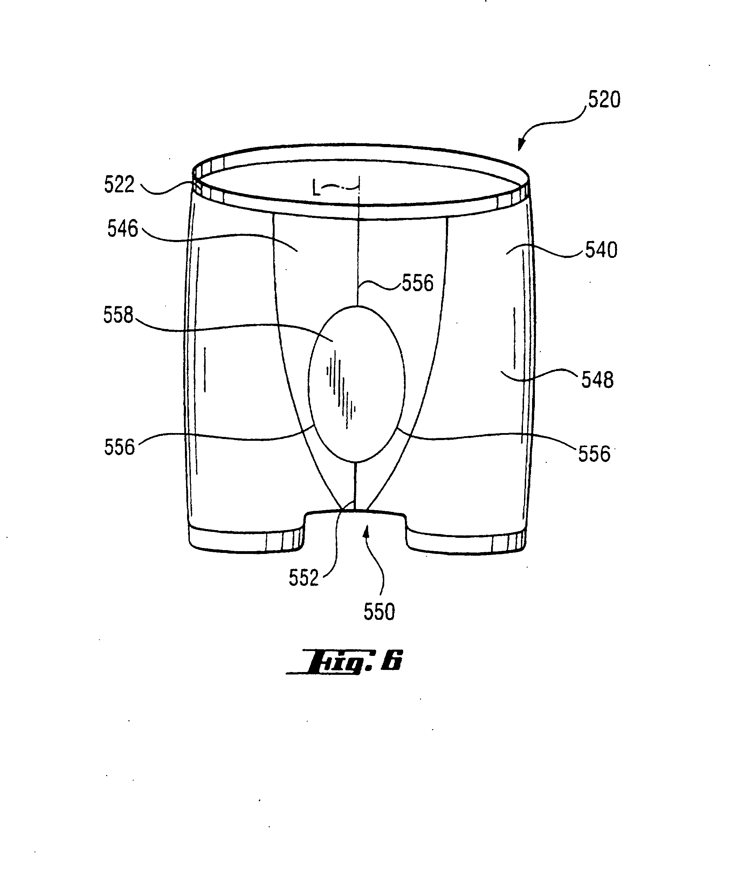

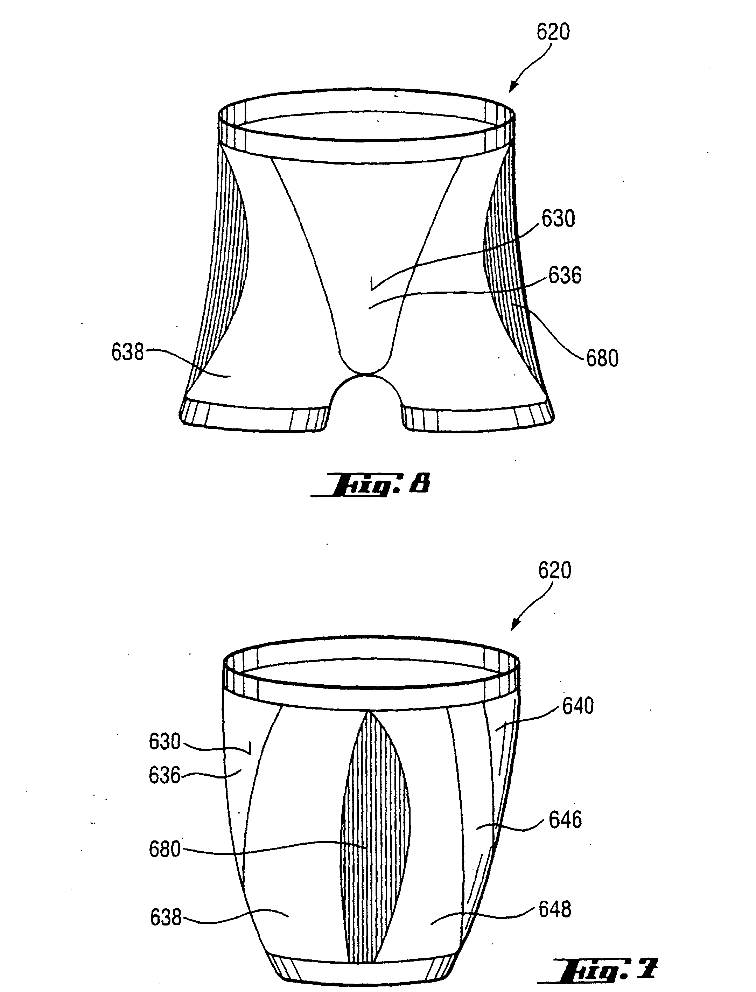

This provides particular benefits versus prior art articles adopting the shape of a "U", or the shape of a W as depicted in Fig. 2b, schematically showing a cross-section through a thin slice of a prior art article 320, with the total absorbent element shaped or folded in this W-form.

The Ω- (Omega)-bunching of the present invention allows to form pockets of longitudinally shape outside of the absorbent member, which can provide certain benefits during use. As the absorbent member will be loaded with at its top "ridge" (230), it will on one side catch the body exudates effectively, as there is only little room for the fluids to go elsewhere. Second, gravity aids - even when high performance materials and structures are used - the penetration of the fluid into the receiving material, thereby minimizing the residence time of the liquid on the surface, and thus the potential for wetting the skin of the wearer or for leaking to the outside.

This advantage becomes even more pronounced, when being combined with core structures having a sub-optimal liquid handling performance. Even if the urine would not penetrate into the article immediately upon contact with the article, it would "run off" along the slopes of the upwardly oriented convex bulge (i.e. the slopes of the Ω [Omega]). If - as might occur with cores of lower performance or capacity - the core is (permanently or momentarily) saturated in this wetted region, fluid that would not penetrate into the core, will be caught by the impermeable sides of the Ω (Omega), which provide good sealing against the outer clothing of the wearer by the liquid impermeable backsheet (216).

A further advantage becomes apparent in figures 2a and 2b, wherein the absorbent core is depicted after it has absorbed significant amounts of urine. This leads to imminent swelling of the core as indicated by the borders after swelling 240 and 242, which, in case of a conventional "W-folded" core, will fill the deeper parts of the W, almost creating a completely filled cup - in particular under the impact of movement of the wearer. In particular for cores comprising superabsorbent materials, which tend to reduce liquid permeability of the matrices they are comprised in, this can lead to the situation where the lower parts of the W (oriented away from the wearer), are hindered in their swelling - be this by geometrical expansion constraint or by lack of fluid transport to these parts.

In case of the present invention, however, the core will swell only the convex bulging of the "Ω", without substantial impeding negatively fluid transport .

If in combination with a high performing core, the functionality of the Ω-bunching with regard to fluid handling changes: now the requirement for the lateral seal becomes less important, but the "Ω-ridge" (230) provides CD-directional flow, which however, will increase the liquid acquisition rate by increasing the loading area in CD-direction, thus also enabling significantly improved longitudinal fluid distribution.

Further, when the article is loaded with feces, these can be deposited into these longitudinally oriented pockets positioned laterally outwardly of the absorbent core, thereby leaving the surface of the absorbent member free for receiving further urinary loading. In particular with relatively thin (or runny, or lower viscosity) feces, these can - e.g. when the wearer is in a standing position due to gravity - flow further down into the crotch region. However, having an upward bulge there, and non-absorbent side sealing, the feces will not cover the absorbent core and not deteriorate its functionality as to receiving urine.

In both instances, the backsheet (216) has to withstand such loading, and must not - even when designed to allow vapors to penetrate through-allow permeation under normal in-use conditions.

As it will also be readily appreciated, these longitudinal side pockets need to be sealed against the skin of the wearer, such as by elastic means (234), positioned between the backsheet (216) and the topsheet (236).

For the scope of the present invention, such pockets may also be formed by so called "leg-cuffs" as well known in the art such as for baby diaper, e.g. in EP-A-0.263.720. In a preferred embodiment of such "leg cuffs", the upstanding part of the secondary cuff is positioned laterally outwardly from the fixed base part of the same cuff, such that it is avoided that these cuffs align on the surface of the absorbent core, and thus reduce the ability to quickly receive even repeated gushes well.

A particularly preferred embodiment for the present invention comprises a chassis whereby these pockets are made by inwardly folding and attaching the backsheet and topsheet material having elastic strands at its longitudinally running edges, such as described in EP-A-0.098.512 (Beckestrøm).

A further element of the present invention results form the transition of the convex bulging in the crotch zone to a concave bulging in the waist zone when being worn.

As is well known from static considerations, arc-like structures have a certain structural strength. This structural strength is an essential element of the present invention in the crotch region, whereby the upward bulging creates such an arc-like structure. As indicated in the above, this convex bulging has to be transformed into a concave structure in the waist region of the article. As can be readily visualized with piece of paper which is folded in one direction in the middle and into the opposite direction at either end, this transition zone has a significant strength and resistance to deformation. In the current context, this results in "pockets" being formed - or void spaces between the article and the body of the wearer - during use.

Such pockets can have a desired effect, as feces can be caught in void spaced formed by the $\Omega$-bunching on the wearer, namely where the convex shape in the crotch region transforms into the concave shape in the rear upper waist part of the article, creating a "rear feces pocket". Such pocket can have a volume suitable for receiving fecal discharges, and comprise from about 10cm$^3$ to about 500cm$^3$ or more.

Such pockets also can have a benefit in a gender specific article design, namely when such a pocket is formed by the front transition zone, thereby forming a space for male genitals.

Such pockets may also be undesired, for example in articles intended for light incontinence use without intention to be loaded with feces, or in the front zone of a genderized female usage product, especially with absorbent cores being relatively stiff by themselves (such as through highly densified cellulosic structures)

In all cases, it will be desirable to have the transition not at an arbitrary position, but rather at the desired location. Henceforth, a preferred embodiment of the present invention includes "hinges" for defined transitioning from the convex to the concave shape.

The relative dimensions of the convex, concave and transition regions depends of course on the intended use and wearer. However, generally human body configurations are such that the lateral extension of the article during use should be small, preferably less than 7 cm, more preferably less than 5 cm, and most preferably even less than about 4 cm. In case of a W-folded core, this dimension relates 4 times the core thickness, in case of the $\Omega$-bunched core, this relates to only 2 times the core thickness (upon neglecting the thickness of the non-core related materials). The length of the convexly bunched region should be at least about 5 cm, to allow adequate contact in the discharge region. The upper limit for this region depends on the intended use, and ranges from only little more than the minimum of 5 cm as describe (e.g. for female, urinary incontinence products) to about 20 cm for articles intended to also receive feces and requiring an "rear feces pocket".

After having described how the various elements of the present invention interact with each other, a more detailed description of the individual features will follow.

Means for sustained $\Omega$ (Omega)-shape

At present, many absorbent articles are produced flat, i.e. having no specific three-dimensional shape. The person who applies the article (i.e. the user him- or herself, or a helping person such as caretaker, parent or the like) can apply the article in various shapes, and by various application steps.

Even if absorbent articles are applied to the wearer such that they form the preferred $\Omega$-shape at least in the crotch zone, normal in-use movements or the loading itself can result in the $\Omega$-shape not being maintained sufficiently pronounced and / or for a sufficiently long period. Thus, in order to maintain the $\Omega$-shape, the article is provided with a means for enhancing maintenance of the $\Omega$-shape during application of the article to the wearer, and also during the use period.

Such a means to provide $\Omega$-bunching can be any means, which is able to deform or to maintain the deformation of the absorbent member in the crotch region such that the above describe shape is formed during application and during use

The most simple way to achieve the Ω-shape during application of the article to the wearer is the folding of the article in the appropriate shape by the manufacturer. Therefore, the article needs to be folded along the longitudinally extending centerline such that the topsheet, which is intended to be oriented to the wearer during use, lies outwardly and the backsheet, which is intended to lie outwardly or towards the clothing of the wearer inwardly of this folded part, which must comprise at least the crotch region, and especially the loading region. With this folding, the person applying the article has a means to readily achieve the Ω-bunched shape upon application.

Alternatively, the article can be folded in any way by the manufacturer, but it comprises a means which just prior to application creates the Ω-shape or supports the Ω-shape when manually created by the person applying the article.

Such means can be elastic features, which - when being in a non-Ω-shape configuration - are stretched, and which contract upon application, thereby forming the Ω-shape, or it can be means for affixing respective backsheets parts to each other.

One way to achieve this is by simply applying adhesive means in a conventional manner similar to the adhesives applied in feminine hygiene products as so called "panty fastening adhesives" for affixing the article to the panty of the wearer. In the present case, however, the objective is to attach backsheet parts not to wearers clothing, but together.

The adhesive can be applied during the manufacturing and then be covered by release papers as well known for the "panty fastening adhesives". The release paper will then be removed at the time of application, just before the respective parts of the backsheet are brought together to form the Ω-shaping. It becomes apparent, that in the first case, packing and shipping of the article is easier, whilst in the second case care must be taken so as to not damage / deteriorate the shaping during transport.

Alternatively, the Ω-shape can be created during manufacturing of the article. In this case, such an adhesive means can be applied to the article when being flat and in an unbulged shape, and the respective parts of the backsheet are then brought together so as to create the bunching.

A further alternative for such an Ω-bunching means is to use mechanical attachment means, such as generally referred to as "mechanical fastening means", whereby a first member being applied to one part (e.g. made of hooks) is mechanically engaging in a second member (e.g. a looped landing zone).

An even preferred alternative for affixing the respective parts of the backsheet together is by not doing so in a firm way but by allowing some relative movement of the backsheet surfaces vs. each other in the longitudinal direction. This relative movement provides particular benefits during the movement of the wearer, such as when walking. Then, the Ω-shaped tunnel will be somewhat "distorted" without, however, loosing its functionality nor its general Ω-shape.

This is particularly useful, if the articles are intended to be worn by mobile persons, such as mobile, non-bed-ridden adults, or toddlers, as it will allow increased comfort during walking.

Such a feature can be one or more elastic bands or stripes (such as indicated with 240 in Fig. 2a) having at least a CD-directional contractional force component. They can be attached towards the lateral edges (i.e. towards the waist regions) of the backsheet but not to the central part (i.e. in the crotch region) of the backsheet, thus pulling together the lateral edges, thus forming the upward bulge or Ω-bunch. Whilst the elastic means for the present invention not necessarily has to be affixed at the outer edges (i.e. the lower base of the "Ω"), care must be taken to shape the article such that only the upper convex part of the Ω is formed by the absorbent core and not a complete W shape is taken, thereby (i.e. to have no absorbent core in the region of the side flaps 225). This can be achieved by the core having a certain stiffness in combination with the structural stiffness resulting from the bunching.

Such elastic elements can be positioned outside the backsheet, or inside, they should however be positioned underneath the absorbent core member (i.e. directed away from the wearer), as otherwise the risk of resulting in a U-shape configuration is too high.

Application of CD-directional stretch features has been disclosed for example in EP-A-0.652.175.

Another preferred alternative can be to position the fixation means in some distance from the backsheet such as by a spacer or block element, thereby forming a hinge between the left and right side of the lateral longitudinal edges of the article.

In another preferred embodiment, not only the backsheet parts are brought into close contact, but also to further bond the total absorbent member through its entire thickness.

This allows that the shape will be maintained better even under stressful in-use conditions.

This bond should, whilst being sufficiently strong to withstand in use stress and also wetting, be sufficiently soft to not increase the discomfort of the wearer.

Other ways to achieve bonds between the backsheets, between backsheet and additonal means for maintaining and sustaining Ω-bunching are other well known techniques such as glue application, melt-bonding, and the like.

A particularly preferred execution in the context of a air-permeable backsheet such as a nonwoven material is the application of pointwise application of hot air thereby melt-bonding certain parts of the structure together.

In a further execution of the present invention, the upwardly oriented convex shaping (i.e. the Ω-shape) of an absorbent member can be sustained by a separate means which creates an upwardly oriented force in the crotch region (i.e. towards the wearer during use).

In a first execution, such a lifting element can be integrated into a unitary article, such as by positioning elastic elements, which have a contractional force component in the longitudinal direction of the article, on the side of the absorbent member, which is during use oriented away from the wearer. Thereby, the elements should be at least in the crotch and/ or loading zone be in the longitudinal centre line zone, i.e. be not too far away from the longitudinal centre line to form the Ω-bulge, which generally can be achieved if these are not less than 2.5 cm offset the centre line in either / or left and right direction.

In a second embodiment, such a lifting element can be in a separate element, which is not integral or unitary with the element of the absorbent core (i.e. the absorbent pad). Such means can be a separate garment, such as a net pant, to be worn over the absorbent article. A particularly preferred garment for such an application is described in EP 1 032 347 A.

Such a suitable garment comprises an elasticized waistband, a front panel having first and second sections, a rear panel having first and second sections, a crotch region disposed between and joining the front panel to the rear panel and a pair of elasticized leg openings. The first section of the front panel has a greater resistance to stretching in the lateral direction than the second section of the front panel. The first section of the rear panel has a greater resistance to stretching in the lateral direction than the second section of the rear panel. The crotch region is provided with a longitudinal stretch control member that is disposed along the longitudinal centerline of the undergarment. The longitudinal stretch control member limits the stretch of the crotch region in the longitudinal direction causing the crotch region to conform to a wearer's skin surface. A front stretch control member is disposed in the front panel and extends from the longitudinal stretch control member to the waistband. A rear stretch control member is disposed in the rear panel and extends from the longitudinal stretch control member to the waistband.

While such a suitable garment can be assembled from materials that may be known to the art as having the requisite mechanical properties, it is preferably knit, such that the mechanical properties of the various components thereof can be provided by a combination of the knit pattern used for a particular component and the yarns that are used. In a particularly preferred embodiment, the longitudinal stretch control member is integrally knit with the crotch region, the front stretch control member is integrally knit with the front panel, and the rear stretch control member is integrally knit with the rear panel.

Further, two or more of the above elements may be combined in the absorbent article. For example, a pre-folded absorbent pad can be held in place by means of the preferred stretchable garment, as described in the above. Or, a non-prefolded pad can be brought into the Ω-shape configuration by an elastic which is in an extended state during shipment, and upon application creates the Ω-shape configuration, and this pad can be held in place by a conventional stretchable garment, such as a pant, without a specific feature for sustaining the Ω-shape, or by a improved Ω-shape supporting garment. Also, the combination of body adhesives with a pre-folded unitary article comprising MD oriented elastification means for providing a lifting force on the crotch or loading zone can be suitable.

Hinge lines

As has been explained in the above, the "convexo-concave" transition should not be positioned arbitrarily, but should happen at a desired place. This can be achieved by including a "hinge means" in the article, and in particular in the absorbent core, if this is the stiffest element of the article.

Without this additional means, this transition will generally occur somewhat outside the crotch region, both in the front and back region, where due to the absence of the restraining legs of the wearer, the Ω-shape can flatten, and thus allows somewhat easier transition, which still might result in undesired deformation or weakening of the structure.

Thus, it becomes clear, that it will be highly beneficial to define the transition region from concave bulging to convex bulging, which can be achieved by creating hinge lines within the absorbent structure.

Such hinge lines can be created by various means, such as cutting, or embossing (i.e. creating high density lines throughout the absorbent, such that the bending at the edges of these high density lines is eased), or low density lines (e.g. created by forming the article with regions having a lower basis weight of materials, but maintaining the same caliper).

The hinge lines to enhance the convexo-concave transition must extend from the outer longitudinal edges of the core or the stiffness determining element of the article into the direction of the longitudinal center line, i.e. the hinge lines have to be arranged such that they have a cross-directional orientation. These hinge lines can be straight lines or curved ones thereby allowing optimization of the specific design of the article, and the anatomy of the intended wearer group. Also the length of the hinge lines can be readily adopted. Also, several independent lines can be used within each one of the transition zones, or one line can split into other. Preferably, such hinge lines do not run through the full width of the absorbent member, as this might result in an undesired loss in strength, or integrity, in particular during use.

Such hinge lines can be created by various means, such as cutting, or embossing (i.e. creating high density lines throughout the stiff member such as the absorbent core, thereby easing the bending at the edges of these high density lines), or low density lines (e.g. as achieved by creating lower basis weight zones of material in the stiff element and densifying it to a lesser degree than the surrounding higher basis weight regions).

Design Capacity and Ultimate Storage Capacity of the absorbent element

In order to be able to compare absorbent articles for varying end use conditions, or differently sized articles, the "design capacity" has been found to be a suitable measure.

For example, babies are representing a typical usage group, but even within this group the amount of urine loading, frequency of loading, composition of the urine will vary widely from smaller babies (new-born babies) to toddlers on one side, but also for example among various individual toddlers.

Another user group may be larger children, still suffering from a certain form of incontinence.

Also, incontinent adults can use such articles, again with a wide range of loading conditions, generally referred to as light incontinence ranging up to severe incontinence.

Henceforth, such articles being able to cope with such requirements should have the capability of picking up such amounts of urine, which will be referred to for the further discussion as "design capacity".

These amounts of fluids have to be absorbed by materials which can ultimately store the bodily fluids, or at least the aqueous parts of these, such that - if any - only little fluid is left on the surface of the article towards the wearers skin. The term "ultimate" refers in one respect to the situation as in the absorbent article at long wearing times, in the other respect to absorbent materials which reach their "ultimate" capacity when being equilibrated with their environment. This can be in such an absorbent article under real in-use conditions after long wearing times, or this also can be in a test procedure for pure materials or material composites. If the processes under consideration have asymptotic kinetic behavior, one skilled in the art will readily consider "ultimate" capacities to be reached when the actual capacity has reached a value sufficiently close to the asymptotic endpoint, e.g. relative to the equipment measurement accuracy.

As an absorbent article can comprise materials which are primarily designed to ultimately store fluids, and other materials which are primarily designed to fulfill other functions such as acquisition and/or distribution of the fluid, but may still have a certain ultimate storage capability, suitable core materials according to the present invention are described without attempting to artificially separate such functions. Nonetheless, the ultimate storage capacity can be determined for the total absorbent core, for regions thereof, for absorbent structures, or even sub-structures, but also for materials as being used in any of the previous.

In case of applying the present invention to other articles requiring different end-uses, one skilled in the art will be able to readily adopt the appropriate design capacities for other intended user groups.

In order to determine or evaluate the Ultimate Design Storage Capacity of an absorbent article, a number of methods have been proposed.

In the context of the present invention, it is assumed, that the Ultimate Storage Capacity of an article is the sum of the ultimate absorbent capacities of the individual elements or material. For these individual components, various well established techniques can be applied as long as these are applied consistently throughout the comparison. For example, the Tea Bag Centrifuge Capacity as developed and well established for superabsorbent polymers can be used for such materials, but also for others (see above).

Once the capacities for the individual materials are known, the total article capacity can be calculated by multiplying these values (in ml/g) with the weight of the material used in the article.

For materials having a dedicated functionality other than ultimate storage of fluids - such as acquisition layers and the like - the ultimate storage capacity can be neglected, either as such materials do in fact have only very low capacity values compared to the dedicated ultimate fluid storage materials, or as such materials are intended to not be loaded with fluid, and thus should release their fluid to the other ultimate storage materials.

With such definitions, for example a so-called "panty liner" product exhibits very low Ultimate storage capacities of a few ml or less. Feminine Hygiene pads have often up to about 20 ml, light urinary incontinence articles have for example 75 ml or about 90ml, medium urinary incontinence articles, or also smaller baby diaper can have about 165 ml, and toddler size baby diapers reaching 300 ml or more, and severe adult incontinence article having 600 ml or more of ultimate storage capacity.

Teabag Centrifuge Capacity Test

The Teabag Centrifuge Capacity test measures the Teabag Centrifuge Capacity values, which are a measure of the retention of liquids in the gelling material at hydrostatic pressure

The superabsorbent material is placed within a "teabag", immersed in a 0.9 % by weight sodium chloride solution for 20 minutes, and then centrifuged for 3 minutes. The ratio of the retained liquid weight to the initial weight of the dry superabsorbent material is the absorptive capacity of the superabsorbent material.

2l of 0.9% by weight sodium chloride in distilled water is poured into a tray having dimensions 24cm x 30 cm x 5cm. The liquid filling height should be about 3cm.

The teabag pouch has dimensions 6.5cm x 6.5cm and is available from a company called Teekanne in Düsseldorf, Germany. The pouch is heat sealable with a standard kitchen plastic bag sealing device (e.g. VACUPACK$_2$ PLUS from Krups, Germany).

The teabag is opened by carefully cutting it partially, and is then weighed. A 0.200g +/- 0.005g sample of the superabsorbent material is placed in the teabag. The teabag is then closed with a heat sealer. This is called the sample teabag.

An empty teabag is sealed and used as a blank.

Each teabag is then held horizontally, and the sample teabag is shaken so as to distribute the superabsorbent material evenly throughout the bag. The sample teabag and the blank teabag are then laid on the surface of the saline solution, and submerged for about 5 seconds using a spatula to allow complete wetting (the teabags will float on the surface of the saline solution but are completely wetted). The timer is started immediately.

After 20 minutes soaking time the sample teabag and the blank teabag are removed from the saline solution, and placed in a Bauknecht WS130, Bosch 772 NZK096 or equivalent centrifuge (230 mm diameter), so that each bag sticks to the outer wall of the centrifuge basket. The centrifuge lid is closed, the centrifuge is started, and the speed increased quickly to 1,400rpm. Once the centrifuge has been stabilized at 1,400rpm the timer is started. After 3 minutes, the centrifuge is stopped.

The sample teabag and the blank teabag are removed and weighed separately.

The Teabag Centrifuge Capacity (TCC) for the sample of superabsorbent hydrogel-forming material is calculated as follows:

TCC = [(sample teabag weight after centrifuging) - (blank teabag weight after centrifuging) - (dry superabsorbent hydrogel-forming material weight)] ÷ (dry superabsorbent material weight).

Garment for holding the absorbent element

The primary means for holding the absorbent element in the appropriate position on the body of the wearer and for achieving and ./or maintaining the Ω-shape at least in the urinary loading zone of the article is a separate garment.

Thereby, the upwardly oriented convex shaping (i.e. the Ω-shape) of an absorbent member can be sustained by a separate means which creates an upwardly oriented force in the crotch region (i.e. towards the wearer during use).

General Description of the Garment

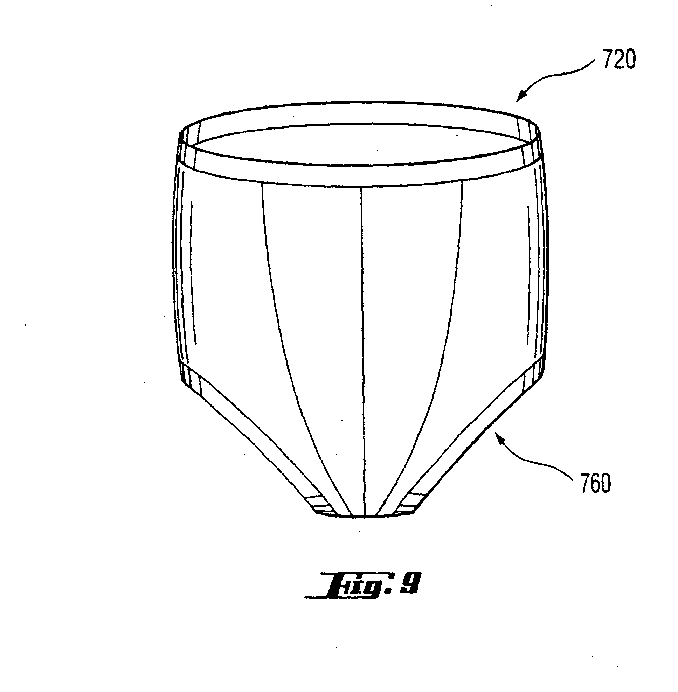

Figures 3 and 4 show front and rear views of the incontinence garment 420 of the present invention. As is shown in Figures 3 and 4, the garment 420 of the present invention comprises a front panel 430 comprising first section 436 and second section 438, a rear panel 440 comprising first section 446 and second section 448, a crotch region 450, a pair of elasticized leg openings 460, and an elasticized waistband 422. The garment 420 is also provided with a waist opening 421 allowing entry into the garment 420. The garment 420 further comprises a longitudinal stretch control member 452 disposed along the longitudinal centerline in the crotch region 450, a front stretch control member 454 disposed in the front panel 430 and extending from the longitudinal stretch control member 452 to the waistband 422, and a rear stretch control member 456 disposed in the rear panel 440 and extending from the longitudinal stretch control member 452 to the waistband 422. Each of these elements will be described in greater detail in the following sections.

Figure 5 shows the garment 420 of the present invention in a full flat out position wherein each of the sides 432, 434 has been opened and elastic components have been pulled flat. Figure 5 can also be considered to be a plan view of a blank for the garment 420 (see Forming the Garment below). As can be seen from 5ure 3, the garment 420 has a longitudinal centerline L and a transverse centerline T. As is also shown clearly in Figure 5, the garment 420 of the present invention is symmetric about the longitudinal axis L and symmetric about the transverse axis T. The garment 420 may also be symmetric about the longitudinal axis L and asymmetric about the transverse axis T.

The garment 420 can comprise woven, nonwoven or knit fabrics. Preferably the garment 420 comprises a knit fabric. A particularly preferred knitting means involves first knitting a seamless tubular blank approximately half the final width of the garment 420. The tubular blank may be knit to have an hour glass shape so as to provide for the leg openings 460 in the finished undergarment 420 or, alternatively, portions of the opened tube may be cut away to provide for such leg openings 460 (see Forming the Garment below).

The Elasticized Waistband

As noted above, the waist opening 421 allows entry into the garment 420 of the present invention. Preferably the waist opening 421 is provided with an elasticized waistband 422 such that the waist opening 421 conforms closely to a wearer's waist. The elasticized waistband 422 may be formed by providing an elastic member, such as a Lycra® or SPANDEX material, adjacent each distal end of the blank that is shown in Figure 5, C-folding each distal end about itself to form end edges 423 and 424, and seaming the distal ends to the front panel 430 and the rear panel 440 to form the waist opening 421 and the elasticized waistband 422. Preferably, the elasticized waistband 422 comprises the same yams as and is integrally knit with the front panel 430 and the rear panel 440. More preferably, the elasticized waistband 422 comprises a turned welt. A particularly preferred knitting pattern for the elasticized waistband 422 comprises a combination of plain knit stitches and float stitches wherein every fourth wale is provided with a positive float stitch.

The Front Panel

As can be seen in Figures 3 and 4, the front panel 430 is that portion of the garment 420 that cooperates with the rear panel 440 (discussed below) to encircle a wearer's waist and hips. As can be also seen in Figures 3 and 4, the front panel 430, the rear panel 440, and the crotch region 450 also cooperate to define the leg openings 460 (discussed in detail below). The front panel 430 comprises first section 436 and second section 438.

While alternate structures can be used, for example, the front panel 430 could be cut to an appropriate shape from a woven or nonwoven material and joined to the remaining portions of the garment 420. The front panel 430 of the present invention is preferably wholly plain knit, more preferably jersey knit, from a combination of elastically extensible and non-elastically extensible yarns. As is clear to one of ordinary skill in the art, the elastic properties of the individual yarns and the particular knitting pattern can be used by a designer to define the mechanical properties of the front panel 430. In a particularly preferred embodiment of the present invention, the front panel 430 comprises alternating courses of wholly plain knit, preferably jersey knit, nylon and Lycra® or SPANDEX yarns as are available from Unifi, Inc. of Greensboro, NC. In an alternative embodiment, the front panel 430 can be wholly plain knit, preferably jersey knit, using a Lycra® or SPANDEX yarn having suitable mechanical properties in all courses. As will be clear from the discussion of the mechanical properties of the front panel 430 below, one of skill in the art could define other knitting patterns using alternative yams to provide such mechanical properties. As noted above, front panels 430 having such mechanical properties comprising woven or nonwoven materials are also envisioned.

In the preferred embodiment of the present invention shown in Figures 3 to 5, the first section 436 has a greater resistance to stretching in the lateral direction than the second section 438. Preferably, the first section 436 also has a greater resistance to stretching in the longitudinal direction than the second section 438. The greater elastic extensibility of the second section 438 enables the garment 420 to fit a variety of body shapes and sizes and provides good conformity to a wearer's body. The greater resistance to stretching of the first section 436, particularly in the lateral direction, provides a "z-direction" biasing force to the absorbent element throughout the full range of wearer movement. Such a biasing force helps maintain the absorbent element worn with the garment 420 in close bodily contact.

The Rear Panel

As mentioned above, the rear panel 440 is that portion of the garment 420 that cooperates with the front panel 430 to encircle a wearer's waist and hips. The rear panel 440 comprises first section 446 and second section 448.

While alternate structures can be used, for example, the rear panel 440 could be cut to an appropriate shape from a woven or nonwoven material and joined to the remaining portions of the garment 420. The rear panel 440 of the present invention is preferably wholly plain knit, more preferably jersey knit, from a combination of elastically extensible and non-elastically extensible yarns. As is clear to one of ordinary skill in the art, the elastic properties of the individual yarns and the particular knitting pattern can be used by a designer to define the mechanical properties of the rear panel 440. In a particularly preferred embodiment of the present invention, the rear panel 440 comprises alternating courses of wholly plain knit, preferably jersey knit, nylon and Lycra® or SPANDEX yarns as are available from Unifi, Inc. of Greensboro, NC. In an alternative embodiment, the rear panel 440 can be wholly plain knit, preferably jersey knit, using a Lycra® or SPANDEX yarn having suitable mechanical properties in all courses. As will be clear from the discussion of the mechanical properties of the rear panel 440 below, one of skill in the art could define other knitting patterns using alternative yarns to provide such mechanical properties. As noted above, rear panels 440 having such mechanical properties comprising woven or nonwoven materials are also envisioned.

In the preferred embodiment of the present invention shown in Figures 3 to 5, the first section 446 has a greater resistance to stretching in the lateral direction than the second section 448. Preferably, the first section 446 also has a greater resistance to stretching in the longitudinal direction than the second section 448. The greater elastic extensibility of the second section 448 enables the garment 420 to fit a variety of body shapes and sizes and provides good conformity to a wearer's body. The greater resistance to stretching of the first section 446 provides a "z-direction" biasing force to the absorbent element throughout the full range of wearer movement. Such a biasing force helps maintain the absorbent element worn with the garment 420 in close bodily contact.

The Crotch Region

The crotch region 450 is positioned along the longitudinal centerline L of the undergarment 420 of the present invention between the front panel 430 and the rear panel 440. In the preferred embodiment of the present invention shown in Figures 3 to 5, the crotch region 450 cooperates with the front panel 430 and the rear panel 440 to define the leg openings 460. As is shown most clearly in Figure 5, a longitudinal stretch control member is disposed along the longitudinal centerline L in the crotch region 450. The crotch region bridges the distance between the elasticized leg openings 460.

While alternate structures can be used, for example, the crotch region 450 could be cut to an appropriate shape from a woven or nonwoven material and joined to the remaining portions of the garment 420. The crotch region 450 of the present invention is preferably wholly plain knit, more preferably jersey knit, from a combination of elastically extensible and non-elastically extensible yarns. As is clear to one of ordinary skill in the art, the elastic properties of the individual yarns and the particular knitting pattern can be used by a designer to define the mechanical properties of the crotch region 450. In a particularly preferred embodiment of the present invention, the crotch region 450 comprises alternating courses of wholly plain knit, preferably jersey knit, nylon and Lycra® or SPANDEX yarns as are available from Unifi, Inc. of Greensboro, NC. In an alternative embodiment, the crotch region 450 can be wholly plain knit, preferably jersey knit, using a Lycra® or SPANDEX yarn having suitable mechanical properties in all courses. As will be clear from the discussion of the mechanical properties of the crotch region 450 below, one of skill in the art could define other knitting patterns using alternative yams to provide such mechanical properties. As noted above, crotch regions 450 having such mechanical properties comprising woven or nonwoven materials are also envisioned.

Preferably the crotch region 450 comprises a knit material having a lower longitudinal stretch modulus than the elasticized leg openings 460 or the longitudinal stretch control member 452. More preferably, as is shown in Figures 3 to 5, the crotch panel 450 is integrally knit with the front panel 430 and the rear panel 440 using a plain knit pattern and yams having a high extensibility.

Longitudinal Stretch Control Member

As noted above the longitudinal stretch control member 452 serves to limit the stretch of the crotch region 450 along the longitudinal centerline L. In particular, the longitudinal stretch control member 452 limits the longitudinally oriented stretch of the crotch region 450 along the longitudinal centerline L. While not being bound by theory, the Applicants believe such longitudinal stretch limitation serves to transfer the "z-direction" biasing force from the rear panel 440 and from the front panel 430 to the crotch region 450. Such force transfer causes the crotch region 450 and any incontinence device 100 disposed thereon to be held closely against a wearer's body (particularly along the longitudinal centerline L of the garment 420) throughout a wide range of wearer movements.

The Applicants have found that the garment 420 of the present invention is particularly comfortable to wear, notwithstanding the close conformity of the present garment to and contact with a wearer's body, particularly in the crotch area as is discussed herein. Garments and/or undergarments of the prior art have attempted to achieve conformity to the crotch area by elasticized lifting members, such as cinches, or by a very tight fit overall. These undergarments are often described as being uncomfortable. One source of such discomfort, particularly for cinch-type undergarments, is pressure on a wearer's anus. The tissue surrounding the anus is particularly sensitive to pressure and forces applied to the anus can cause discomfort. Cinch-type undergarments, such as that described in U.S. Patent 3,608,551, typically use an elastically extensible member to provide a lifting force to seal an absorbent article against a wearer's perineum. Such elastic members are usually joined to the undergarment at a location that is positioned above a wearer's anus when the undergarment is wom. As a result, there is not only the desirable lifting force to seal an absorbent article against the wearer's perineum but also an uncomfortable pressure on a wearer's anus. On the other hand, the garment 420 of the present invention distributes the "z-direction" biasing force discussed above so that bodily contact is maintained throughout a wide range of wearer motions without unacceptable pressure on a wearer's anus.

As shown most clearly in Figure 5, the longitudinal stretch control member 452 is disposed along the longitudinal centerline L in the crotch region 450. The longitudinal stretch control member 452 can be either a separate element joined to the crotch region 450 or it can be integral to the crotch region 450. Preferably, the longitudinal stretch control member 452 is integral to the crotch region 450. In a particularly preferred embodiment of the present invention, the longitudinal stretch control member 452 and the crotch region 450 are integrally knit.

As noted above, the longitudinal stretch control member 452 serves to limit stretch, particularly longitudinally oriented stretch in the crotch region 450 along the longitudinal centerline L. To this end, the longitudinal stretch control member 452 can comprise any material having a greater stretch modulus than the crotch region 450. For example, the longitudinal stretch control member 452 could comprise a high modulus film material or even a single strand of yarn or monofilament having a relatively high modulus. For the preferred integrally knit longitudinal stretch control member 452, the longitudinal stretch control member could comprise the same yarns used for the crotch region wherein the yams comprising the stretch control member 452 were knit in a pattern known to the art as being stretch limiting. For example, the longitudinal stretch control member 452 can comprise a knit pattern wherein alternating courses thereof are tucked. Alternatively, an elastic yarn can be floated in to provide the longitudinal stretch control member 452 with additional stretch resistance as is also known in the art.

Suitable yarns for the longitudinal stretch control member 452 are substantially the same yarns or combinations of yarns as have been found to be suitable for the crotch region 450.

The longitudinal stretch control member 452 has a greater resistance to stretching in the longitudinal direction than said first section 436 of said front panel 430. The longitudinal stretch control member 452 has a greater resistance to stretching in the longitudinal direction than said first section 446 of said rear panel 440.

The Front Stretch Control Member

The front stretch control member 454 cooperates with the longitudinal stretch control member 452 to provide a "z-direction" biasing force along the longitudinal centerline L of the garment 420 particularly in the crotch region 450. This force helps lift the crotch region 450, particularly the longitudinal stretch control 452 member that is disposed therein, so that any absorbent element that may be disposed thereon is in close body contact. In particular, the Applicants believe that the front stretch control member 454 directs the forces provided by the longitudinal stretch control member 452 to the waistband 422 to help lift the crotch region 450 into close bodily contact.

As noted above, the front stretch control member 454 helps provide "z-direction" biasing force along the longitudinal centerline L. Therefore, the front stretch control member 454 is preferably disposed along the longitudinal centerline L in the front panel 430. More preferably, the front stretch control member 454 divides the first section 436 of the front panel 430 into two identical sections. The front stretch control member 454 can be joined to the front panel 430 along the longitudinal centerline L. Preferably, the front stretch control member 454 is integral to the front panel 430. In the particularly preferred embodiment shown in Figures 3 to 5, the front stretch control member 454 is integrally knit with the first section 436 of the front panel 430.

To facilitate the direction of forces, the front stretch control member 454 should have less stretch than the first and second sections 436, 438 of the front panel 430. To provide such lower stretch, the front stretch control member 454 may comprise a material having a higher stretch modulus than the front panel 430 or a knit material having a knit pattern as is known in the art to provide greater stretch resistance. Higher stretch modulus materials suitable for use as a front stretch control member 454 include high modulus film materials, such as a polyester film material or even a single strand of yarn or monofilament having a relatively high modulus (e. g. cotton, polyester or nylon). Preferably, the front stretch control member 454 comprises the same yarns as are suitable for the first and second sections 436, 438 of the front panel 430 and is integrally knit therewith using a knit pattern having less stretch than the first and second sections 436, 438. That is, the yarns discussed above with respect to the first and second sections 436, 438 of the front panel 430 are also suitable for the front stretch control member 454. A particularly preferred knitting pattern for the front stretch control member 454 uses stitches known in the art to provide reduced stretch. For example, a pattern of tuck stitches has been found to be suitable.

The Rear Stretch Control Member

The rear stretch control member 456 cooperates with the longitudinal stretch control member 452 to provide a "z-direction" biasing force. This force helps lift the crotch region 450, particularly the longitudinal stretch control 452 member that is disposed therein, so that any absorbent element that may be disposed thereon is in close body contact. In particular, the Applicants believe that the rear stretch control member 456 directs the forces provided by the longitudinal stretch control member 452 to the waistband 422 to help lift the crotch region 450 into close bodily contact.

As noted above, the rear stretch control member 456 helps provide a "z-direction" biasing force. The rear stretch control member 456 preferably extends from the longitudinal stretch control member 452 along two lines spaced from the longitudinal centerline L in the rear panel 440. By spacing the rear stretch control member 456 from the longitudinal centerline L, the high forces of the rear stretch control member 456 are diverted away from the longitudinal centerline L. Applicants have found this to be particularly important as this allows the first section 446 of the rear panel 440 to be positioned over the anus. Since the first section 446 has a lower resistance to stretch in both the longitudinal and lateral directions than the rear stretch control member 456, the first section is able to expand under lower forces. This zone of lower force expansion creates a pocket 458 in the rear panel 440 which can expand to contain BM.

The rear stretch control member 456 can be joined to the rear panel 440. Preferably, the rear stretch control member 456 is integral to the rear panel 440. In the particularly preferred embodiment shown in Figures 3 to 5, the rear stretch control member 456 is integrally knit with the first section 446 of the rear panel 440.

To facilitate the direction of forces, the rear stretch control member 456 should have less stretch than the first and second sections 446, 448 of the rear panel 440. To provide such lower stretch, the rear stretch control member 456 may comprise a material having a higher stretch modulus than the rear panel 440 or a knit material having a knit pattern as is known in the art to provide greater stretch resistance. Higher stretch modulus materials suitable for use as a rear stretch control member 456 include high modulus film materials, such as a polyester film material or even a single strand of yarn or monofilament having a relatively high modulus (e. g. cotton, polyester or nylon). Preferably, the rear stretch control member 456 comprises the same yarns as are suitable for the first and second sections 446, 448 of the rear panel 440 and is integrally knit therewith using a knit pattern having less stretch than the first and second sections 446, 448. That is, the yarns discussed above with respect to the first and second sections 446, 448 of the rear panel 440 are also suitable for the rear stretch control member 456. A particularly preferred knitting pattern for the rear stretch control member 456 uses stitches known in the art to provide reduced stretch. For example, a pattern of tuck stitches has been found to be suitable.

Elasticized Leg Openings

As can be seen in Figures 3 to 5, the garment 420 of the present invention is also provided with a pair of elasticized leg openings 460. As noted above, the front panel 430, the rear panel 440, and the crotch region 450 cooperate to define the periphery of each leg opening 460. This periphery is provided with a leg elastic 462 for elasticization of the leg opening 460. The leg elastics 462 both provide a seal against leakage of bodily fluids about the periphery of each leg.

While the leg elastics 462 must provide a minimal contractive force help to seal the periphery of the leg opening 460 against leakage of bodily fluids, it is important that the contractive force not be so great as to cause discomfort to a wearer. Minimizing the stretch modulus over the range of expected elastic extensions during the wear cycle also minimizes the risk of wearer discomfort. That is, if the leg elastics are designed to provide a contractive force at a typical in use extension, that force should not substantially increase for greater extensions that may either be due to a different wearer leg circumference or due to wearer movement.

The leg elastics 462 can be joined to the front panel 430, the rear panel 440, and the crotch region 450 about the periphery of the leg opening 460 using means known to those of skill in the art. Specifically, the leg elastics 462 are joined to that portion of the side edges 425, 426, 427, 428 which will surround the leg openings 460 (i. e. form the periphery thereof). For example, the leg elastics 462 can be joined to the front panel 430, the rear panel 440, and the crotch region 450 using adhesive means or by mechanical means, such as stitching. For the preferred knit garment 420 of the present invention, the leg elastics 462 are preferably joined to the front panel 430, the rear panel 440, and the crotch panel 450 by stitching thereto.

Forming the Undergarment

A blank for the garment 420 is first knit in a tubular form using means known to the art. In particular, front panel 430, the rear panel 440, the crotch region 450 are integrally knit. The first section 436 of the front panel 430 is provided with a front stretch control member 454 by having such a strip integrally knit therein. The first section 446 of the rear panel 440 is provided with a rear stretch control member 456 by having such a strip integrally knit therein. Similarly, the crotch region 450 is provided with an integrally knit longitudinal stretch control member 452. The appropriate knit patterns as described above are used.

The tubular blank is then slit walewise and opened. Excess material that would otherwise fill the leg openings 460 is removed to form a flat blank for the garment 420 having a shape similar to the plan view of the garment 420 that is shown in Figure 5. As is further shown in Figure 5, the blank for the garment has a front end edge 423, a rear end edge 424, front side edges 425, 426, and rear side edges 427, 428.

The leg elastics 462 are joined to the garment 420 about the periphery of the leg openings 460 as discussed above. The blank for the garment 420 is then folded about the transverse centerline T and opposing portions of the side edges that lie between the leg opening 460 and the end edges 423, 424 are joined to form side seams 432, 434 completing the assembly of garment 420 (That is, the portion of side edge 425 that lies between the end of the leg elastic 462 in front panel 430 and the end edge 424 is joined to the portion of side edge 427 that lies between the end of the leg elastic 462 that lies in the rear panel 440 and the end edge 423 to form seam 432. Side edge 426 is joined to side edge 428 in a similar manner to form seam 434).

Alternatively, portions of the tubular knit blank can be cut out to provide the leg openings 460. For example, a tubular blank can be flattened, such that, the interior faces thereof contact each other and a pair longitudinally oriented side edges are formed. Leg opening precursors can then be formed by cutting matching portions having a semi-circular, semi-elliptical, or other desired shape from transversely opposite side edges at regular intervals along the flattened blank. Garment blanks are then formed by transversely cutting the flattened tubular blank in a predetermined repeat pattern wherein a first transverse cut is made across the material that was not removed when the leg opening precursors were formed to create a crotch portion precursor and a second transverse cut is made across the full width of the flattened tubular blank forming the waist opening 421. The leg elastics 462 are disposed about the periphery of each leg opening 460 and joined thereto. The two ends formed by the first transverse cut are joined by a single transverse seam to complete the crotch region 450. The garment 420 is then finished by disposing the elasticized waistband 422 about the periphery of the waist opening 421 and joining the elasticized waistband 422 thereto.

Alternative Embodiments

Referring now to Figure 6 there is shown a rear view of an alternative embodiment of a garment 520 of the present invention. Garment 520 is identical to garment 420 except for the configuration of the rear stretch control member 556.

The rear stretch control member 556 cooperates with the longitudinal stretch control member 552 to provide a "z-direction" biasing force. This force helps lift the crotch region 550, particularly the longitudinal stretch control 552 member that is disposed therein, so that an absorbent element that may be disposed thereon is in close body contact. In particular, the Applicants believe that the rear stretch control member 556 directs the forces provided by the longitudinal stretch control member 552 to the waistband 522 to help lift the crotch region 550 into close bodily contact.

As noted above, the rear stretch control member 556 helps provide a "z-direction" biasing force. The rear stretch control member 556 preferably extends from the longitudinal stretch control member 552 along two arcuate lines spaced from the longitudinal centerline L along a portion of their length and then converge to form a single line along the longitudinal centerline L prior to reaching the waistband 522. By spacing the rear stretch control member 556 from the longitudinal centerline L at least along a portion of its length, the high forces of the rear stretch control member 556 are diverted away from the longitudinal centerline L creating a pocket 558. Applicants have found this to be particularly important as the design of the garment 520 positions the pocket 558 over the anus. Since the pocket 558 has a lower resistance to stretch in both the longitudinal and lateral directions than the rear stretch control member 556, the pocket 558 is able to expand under lower forces. This zone of lower force expansion creates a pocket 558 in the rear panel 540 which can expand to contain BM.

The rear stretch control member 556 can be joined to the rear panel 540. Preferably, the rear stretch control member 556 is integral to the rear panel 540. In the particularly preferred embodiment shown in Figure 6, the rear stretch control member 556 is integrally knit with the first section 546 of the rear panel 540.

To facilitate the direction of forces, the rear stretch control member 556 should have less stretch than the first and second sections 546, 548 of the rear panel 540. To provide such lower stretch, the rear stretch control member 556 may comprise a material having a higher stretch modulus than the rear panel 540 or a knit material having a knit pattern as is known in the art to provide greater stretch resistance. Higher stretch modulus materials suitable for use as a rear stretch control member 556 include high modulus film materials, such as a polyester film material or even a single strand of yarn or monofilament having a relatively high modulus (e. g. cotton, polyester or nylon). Preferably, the rear stretch control member 556 comprises the same yarns as are suitable for the first and second sections 546, 548 of the rear panel 540 and is integrally knit therewith using a knit pattern having less stretch than the first and second sections 546, 548. That is, the yarns discussed above with respect to the first and second sections 546, 548 of the rear panel 540 are also suitable for the rear stretch control member 556. A particularly preferred knitting pattern for the rear stretch control member 456 uses stitches known in the art to provide reduced stretch. For example, a pattern of tuck stitches has been found to be suitable.

Referring now to Figures 7 and 8 there is shown an alternative embodiment of a garment 620 of the present invention. Garment 620 is identical to garment 420 except for the addition of the side panels 680.

As can be seen in Figures 7 and 8, the side panel 680 is that portion of the garment 620 that cooperates with the front panel 630 and the rear panel 640 to encircle a wearer's waist and hips. More specifically, the side panel 680 is that portion of the garment 620 that joins the second section 638 of the front panel 630 with the second section 648 of the rear panel 640.

While alternate structures can be used, for example, the side panel 680 could be cut to an appropriate shape from a woven or nonwoven material and joined to the remaining portions of the garment. The side panel 680 is preferably wholly plain knit, more preferably jersey knit, from a combination of elastically extensible and non-elastically extensible yarns. As is clear to one of ordinary skill in the art, the elastic properties of the individual yarns and the particular knitting pattern can be used by a designer to define the mechanical properties of the side panel 680. In a particularly preferred embodiment of the present invention, the side panel 680 comprises alternating courses of wholly plain knit, preferably jersey knit, nylon and Lycra® or SPANDEX yarns as are available from Unifi, Inc. of Greensboro, NC. In an alternative embodiment, the side panel 680 can be wholly plain knit, preferably jersey knit, using a Lycra® or SPANDEX yarn having suitable mechanical properties in all courses. As will be clear from the discussion of the mechanical properties of the side panel 680 below, one of skill in the art could define other knitting patterns using alternative yams to provide such mechanical properties. As noted above, side panels 680 having such mechanical properties comprising woven or nonwoven materials are also envisioned.

In the embodiment shown in Figures 7 and 8, the side panel 680 has a greater resistance to stretching in the lateral direction than the second section 638 of the front panel 630 and the second section 648 of the rear panel 640. Preferably, the side panel 680 has a greater resistance to stretching in the longitudinal direction than the second section 638 of the front panel 630 and the second section 648 of the rear panel 640.

In the embodiment shown, the first section 636 of the front panel 630 has a greater resistance to stretching in the lateral direction than the side panel 680. The first section 646 of the rear panel 640 has a greater resistance to stretching in the lateral direction than the side panel 680. The first section 636 of the front panel 630 has a greater resistance to stretching in the longitudinal direction than the side panel 680. The first section 646 of the rear panel 640 has a greater resistance to stretching in the longitudinal direction than the side panel 680.

During use it is preferred that the second sections 638 and 648 stretch first as they provide the least resistance to stretch. If the wearing forces are increased, the side panels 680 should stretch next with the last portions of the pant to expand being the first sections 636 and 646. This is preferred as first sections 636 and 646 are intended to maintain the incontinence pad in place while the other sections, second sections 638 and 648 and side panels 680, are intended to provide close body fit.

Referring now to Figure 4, there is shown a preferred embodiment of a rear stretch control member 456 which extends from the longitudinal stretch control member 452 along two lines spaced from the longitudinal centerline L in the rear panel 440. A similar construction of the front stretch control member 454 to that of the rear stretch control member 456 shown in Figure 4, i.e., having the front stretch control member 454 extend from the longitudinal stretch control member 452 along two lines spaced from the longitudinal centerline L, may be advantageous for the male user to provide a lower force region in the garment adjacent the male genitalia. Similarly, the front stretch control member may be constructed similar to the rear stretch control member 556 shown in Figure 6 to provide a pocket for the male genitalia which has a lower resistance to stretch than the stretch control member.

Referring now to Figure 9 there is shown an alternative embodiment of a garment 720 of the present invention. Garment 720 is identical to garment 20 except for the configuration of the elasticized leg openings 760. As can be seen in Figure 10 the elasticized leg openings 760 are cut higher compared to elasticized leg openings 460 shown in Figure 3. The higher cut of elasticized leg openings 760 provides a bigger opening than leg openings 460. In addition, garment 720 uses less overall material than garment 20.

Other means for achieving and maintaining Ω (Omega)-shape

At present, many absorbent articles are produced flat, i.e. having no specific three-dimensional shape. The person who applies the article (i.e. the user him- or herself, or a helping person such as caretaker, parent or the like) can apply the article in various shapes, and by various application steps.

Even if absorbent articles are applied to the wearer such that they form the preferred Ω-shape at least in the crotch zone, normal in-use movements or the loading itself can result in the Ω-shape not being maintained sufficiently pronounced can be for a sufficiently long period. Thus, in order to maintain the Ω-shape, the article is provided with a means for enhancing maintenance of the Ω-shape during application of the article to the wearer, and also during the use period.

Such a means to provide Ω-bunching can be any means, which is able to deform or to maintain the deformation of the absorbent member in the crotch region such that the above describe shape is formed during application and during use.

The most simple way to achieve the Ω-shape during application of the article to the wearer is the folding of the article in the appropriate shape by the manufacturer. Therefore, the article needs to be folded along the longitudinally extending centerline such that the topsheet, which is intended to be oriented to the wearer during use, lies outwardly and the backsheet, which is intended to lie outwardly or towards the clothing of the wearer inwardly of this folded part, which must comprise at least the crotch region, and especially the loading region. With this folding, the person applying the article has a means to readily achieve the Ω-bunched shape upon application.

Alternatively, the article can be folded in any way by the manufacturer, but it comprises a means which just prior to application creates the Ω-shape or supports the Ω-shape when manually created by the person applying the article.

Such means can be elastic features, which - when being in a non-Ω-shape configuration - are stretched, and which contract, thereby forming the Ω-shape, or it can be means for affixing respective backsheets parts to each other.

One way to achieve this is by simply applying adhesive means in a conventional manner similar to the adhesives applied in feminine hygiene products as so called "panty fastening adhesiveÓ for affixing the article to the panty of the wearer. In the present case, however, the objective is to attach backsheet parts not to wearers clothing, but together.

The adhesive can be applied during the manufacturing and then be covered by release papers as well known for the "panty fastening adhesives". The release paper will then be removed at the time of application, just before the respective parts of the backsheet are brought together to form the Ω-shaping.

Alternatively, the Ω-shape can be created during manufacturing of the article. In this case, such an adhesive means can be applied to the article when being flat and in an unbulged shape, and the respective parts of the backsheet are then brought together so as to create the bunching.

A further alternative for such an Ω-bunching means is to use mechanical attachment means, such as generally referred to as "mechanical fastener", whereby a first member being applied to one part (e.g. made of hooks) which is mechanically engaging in a second member (e.g. a looped landing zone).

An even preferred alternative for affixing the respective parts of the backsheet together is by not doing so in a firm way but by allowing some relative movement of the backsheet surfaces vs. each other in the longitudinal direction. This relative movement provides particular benefits during the movement of the wearer, such as when walking. Then, the Ω-shaped tunnel will be somewhat "distorted" without, however, loosing its functionality nor its general Ω-shape.

This is particularly useful, if the articles are intended to be worn by mobile persons, such as mobile, non-bed-ridden adults, or toddlers, as it will allow increased comfort during walking.

Such a feature can be one or more elastic bands or stripes (such as indicated with 240 in Fig. 2a) having at least a CD-directional contractional force component. They can be attached towards the lateral of the backsheet but not to the central part of the backsheet, thus pulling together the lateral edges, thus forming the upward bulge or Ω-bunch. Whilst the elastic means for the present invention not necessarily has to be affixed at the outer edges (i.e. the lower base of the "Ω"), care must be taken to shape the article such that only the upper convex part of the Ω is formed by the absorbent core and not a complete W shape is taken, (i.e. to have no absorbent core in the region of the side flaps 225). This can be achieved by the core having a certain stiffness in combination with the structural stiffness resulting from the bunching.

Such elastic elements can be positioned outside the backsheet, or inside, they should however be positioned underneath the absorbent core member (i.e. directed away from the wearer), as otherwise the risk of resulting in a U-shape configuration is too high.

Application of CD-directional stretch features has been disclosed for example in CM586 (Festooning).

The elastic elements can have an essential CD orientation, or can also have and MD orientation, such as when being attached more towards the longitudinal edges of the element, i.e. towards the waist regions.

Another preferred alternative can be to position the fixation means in some distance from the backsheet such as by a spacer or block element, thereby forming a hinge between the left and right side of the lateral longitudinal edges of the article.

In another preferred embodiment, not only the backsheet parts are brought into close contact, but also to further bond the total absorbent core or even absorbent element through its entire thickness. This allows that the shape will be maintained better even under stressful in-use conditions. This bond should, whilst being sufficiently strong to withstand in use stress and also wetting, be sufficiently soft to not increase the discomfort of the wearer.

Ways to achieve bonds between the backsheets, between backsheet and additonal means for maintaining and sustaining Ω-bunching are other well known techniques such as glue application, melt-bonding, and the like.

A particularly preferred execution in the context of a air-permeable backsheet such as a nonwoven material is the application of pointwise application of hot air thereby melt-bonding certain parts of the structure together, as described in European Patent Application.

Combination features of the absorbent elements and the garment

The synergistic effects of a absorbent element and a holding garment which are designed to support each other in an improved manner can be more pronounced by combining several features and by specifically adapting design features for each of the elements.

Thus, two or more of the above elements may be combined in the absorbent article. For example, a pre-folded absorbent pad can be held in place by means of the preferred stretchable garment, as described in the above. Or, a non-prefolded pad can be brought into the Ω-shape configuration by an elastic which is in an extended state during shipment, and upon application creates the Ω-shape configuration, and this pad can be held in place by a conventional stretchable garment, such as a pant, without a specific feature for sustaining the Ω-shape, or by a improved Ω-shape supporting garment. Also, the combination of body adhesives with a pre-folded unitary article comprising MD oriented elastification means for providing a lifting force on the crotch or loading zone can be suitable.

Also, either the garment or the absorbent element or both can comprise means for reliably securing the elements to each other. For example, absorbent pad could be provided with a first portion of a cohesive material and the crotch region of the garment could be provided with a second portion of a cohesive material. As used herein, a "cohesive material" is one which preferentially adheres to itself and not to other materials. Such attachment systems are described in U.S. Patent 5,415,650 which issued to Sigl on May 16, 1995.

Alternatively, a "hook and loop" fastening system can be used wherein the garment surface of the absorbent element could be provided with a hook material. For example, a prong made according to U.S. Patent 5,058,247, which issued to Thomas, et al. on October 22, 1991, would be a satisfactory hook material. The crotch region of the garment could be provided with a loop material as is known to the art or, preferably, the yams and/or knitting pattern used for the crotch region could be modified according to the art to provide loops for engaging a hook material.

The crotch region of the garment can also optionally be provided with indicia to help a wearer optimally position the absorbent element therein. For example, such indicia could comprise markings along the longitudinal centerline L that would allow a wearer to reliably position an absorbent element each time a new device is disposed on the body contacting (i.e. inner) surface of the crotch region. In addition, the front panel and the rear panel can also optionally be provided with indicia to help a wearer optimally position an absorbent element therein.

TEST METHODS

Stretch Modulus and Elastic Contractions

Intent

This method is intended to quantify a force comparable to the force exerted on a wearer's body by extensible materials that may be used in an undergarment over an extension range similar to that seen in the wear cycle of an undergarment.

Method

The method described in INDA (Association of Nonwoven Fabric Industry) Standard Test 110.1-92 is suitable. The following set up conditions are used:

| | |
|---|---|
| Gage Length: | 2 inches (5.08 centimeters) |
| Crosshead Speed: | 10 inches/minute (25.4 centimeters/minute) |
| Tensile Testing Machine: | Appropriate for expected force range, a Model |
| and Load Cell | 5564, available from Instron Corporation, Canton, MA is suitable |
| Sample Width: | 1 inch (2.54 centimeters) For samples less than 1 inch (2.54 centimeters) wide, measure the sample width and adjust the measured force by the ratio of 1 inch (2.54 centimeters) to the measured width. |
| Sample Direction: | Longitudinal stretch modulus samples are cut so the sample width is perpendicular to the longitudinal direction. Lateral stretch modulus samples are cut so the sample width is perpendicular to the lateral direction. |
| Sample Size: | At least three samples per material tested |

Calculations

| | |
|---|---|
| $Force_0$: | Force at start of data collection (grams/inch |
| | or grams/cm) Is there a prestretch before |
| | starting to take data |
| $Force_{25}$: | Force at 25% elongation (grams/inch or grams/cm) |

(continued)

| |
| --- |
| Elastic Contractions = $Force_{25}$ |
| Stretch Modulus = $(Force_{25} - Force_0)/0.25$ |

Report the mean and standard deviation for elastic contractions (leg elastics only) and for stretch modulus

**Claims**

1. Absorbent article comprising an absorbent element comprising a unitary loading zone and a garment (420) for holding said absorbent element, wherein said absorbent element comprises a convex bulging shape of the absorbent core (10) oriented upwardly towards the wearer at least for parts of said uritary loading zone,
   and wherein said garment (420) comprises a longitudinal stretch control member disposed along the longitudinal centerline of the article at least in the urinary loading zone in registry with the convexly bulged zone,
   **characterized in that** said convex bulging shape at least of the urinary loading zone is achieved by means of a fold of the absorbent element along the longitudinal centerline of the article at least in said urinary loading zone such that at least parts of the backsheet covering the absorbent core are in direct contact with each other.

2. Absorbent article according to claim 1, wherein
   said garment (420) has
   a longitudinal centerline defining a longitudinal direction and a lateral centerline defining a lateral direction, said garment comprising:

   an elasticized waistband (422);
   a front panel (430) having first and second sections (436,438), said first section (436) having a greater resistance to stretching in the lateral direction than that of said second section (438);
   a rear panel (440) having first and second sections (446,448), said first section having
   a greater resistance to stretching in the lateral direction than that of said second section;
   a crotch region (450) disposed between and joining said front panel to said rear panel;
   a pair of elasticized leg openings (460);
   a longitudinal stretch control member (452) disposed along said longitudinal
   centerline in said crotch region, said longitudinal stretch control member (452) serving to limit the extent of longitudinally oriented stretch of said crotch region along the longitudinal centerline; a front stretch control member (454) disposed in said front panel (430) and
   extending from said longitudinal stretch control member (452) to said waistband to direct the forces from said longitudinal stretch control member to said waistband; and
   a rear stretch control member (456) disposed in said rear panel (440) and extending from said longitudinal stretch control member to said waistband (422) to direct the forces from said longitudinal stretch control member to said waistband.

3. A garment (420) according to Claim 1 wherein said first section (436;446) of said front or rear panel has a greater resistance to stretching in the longitudinal direction than that of said second section (438;448) of said respective front or rear panel.

4. A garment (420) according to Claim 1 wherein said longitudinal stretch control member (452) has a greater resistance to stretching in the longitudinal direction than that of the respective first section (436;446) of said front panel or of said rear panel.

5. A garment (420) according to Claim 1 wherein said first and second sections of said front or rear panel have a longitudinal stretch modulus, said longitudinal stretch modulus of said first section (436-446) being greater than said longitudinal stretch modulus of said second section (438-448) of said front or rear panel.

6. A garment (420) according to Claim 1 wherein said first and second sections of said front or rear panel have a lateral stretch modulus, said lateral stretch modulus of said first section (436;446) being greater than said lateral stretch modulus of said second section (438;448) of said front or rear panel.

**7.** A garment according to Claim 1 wherein said garment (420) comprises a knit material.

**8.** A garment (420) according to Claim 1 further comprising a side panel joining said first panel to said rear panel (440).

**9.** A garment (420) according to Claim 8 wherein said side panel has a greater resistance to stretching in the lateral direction than that of said second section of said front or said rear panel (440).

**10.** A garment (420) according to Claim 8 wherein said first section (436; 446) of said front or said rear panel has a greater resistance to stretching in the lateral direction than that of said side panel.

**11.** Absorbent article according to claim 1, further **characterized in that** said means for maintaining the absorbent core (10) in the crotch region in a convex bulging shape is an adhesive or meachanical fastening means applied outwardly on the backsheet (16) of the absorbent element.

**12.** Absorbent article according to any of claims 1 or 2, further **characterized in that**
said means for maintaining the core in the crotch region in a convex bulging shape is an elastically contractible element with at least a CD-directional force component

**13.** Absorbent article according to any of claim 1, further **characterized in that** it has a length and a width dimension, and a z-direction perpendicular to both these dimensions,
further comprising a second means which is unitary with the absorbent element for maintaining the absorbent core (10) in the urinary loading zone in a convex bulging shape which is an elastically contractible element with at least an MD-directional force component at least exerting a z-directional force component oriented towards the wearer during use.

**14.** Absorbent article according to any of claims 1 to 14, further **characterized in that** it further comprising one or more convexo-concave transition means positioned between the urinary loading zone and the one or two waist region(s) of the absorbent element.

**15.** Absorbent article according to claim 15 further **characterized in that** said convexo-concave transition means is a or are hinge line(s).

**16.** Absorbent article according to any of claims 1 to 16 further characterrized in that said urinary loading zone is designed to maintain convex bunching of the absorbent element for at least 50 mm length during application and during use.

**17.** Absorbent article according to any of claims 1 to 17 further **characterized in that** the article further comprises fixation means of the type being of adhesives being topically applied to the skin of the wearer for fixation of the article on the body of the wearer.

**18.** Absorbent article according to any of the preceding claims, further **characterized in that**
a void is formed in use between the urinary loading zone having a convex shaping with at least one of the concavely shaped waist zones and the body of the wearer during use.

**19.** Absorbent article according to claim 1,
wherein said absorbent element and said garment (420) are affixed to each other by one or more attachment means.

**20.** Absorbent article according to claim 20 wherein at least one of said attachment means is an adhesive bond between the two elements for maintaining the convex bulging at least in the urinary loading zone.

**21.** Absorbent article according to claim 21
wherein at least one of said attachment means is an releasable adhesive or mechanical fastening means.

**22.** Absorbent article according to claim 24, wherein at least one of said attachment means is positioned along the longitudinal centerline of both elements.

**EP 1 033 959 B1**

**Patentansprüche**

1. Absorbierender Artikel umfassend ein absorbierendes Element mit einer Urin-Aufnahmezone und ein Kleidungsstück (420) zum Halten des absorbierenden Elementes, wobei das absorbierende Element eine konvexe ausgebauchte Form des absorbierenden Kerns (10) aufweist, der nach oben zu dem Träger mindestens in Abschnitten der Urin-Aufnahmezone verläuft,
und wobei das Kleidungsstück (420) ein Längsdehnungs-Steuerungselement aufweist, das entlang der Längs-Mittellinie des Artikels mindestens in der Urin-Aufnahmezone in Ausrichtung mit der konvexausgebauchten Zone angeordnet ist, **dadurch gekennzeichnet, dass** die konvexe ausgebauchte Form von mindestens der Urin-Aufnahmezone eine Falte des absorbierenden Artikels entlang der Längs-Mittellinie des Artikels mindestens in der Urin-Aufnahmezone erreicht wird, so dass mindestens den absorbierenden Kern überdeckende Abschnitte der Außenlage in direktem Kontakt miteinander stehen.

2. Absorbierender Artikel nach Anspruch 1, wobei das Kleidungsstück (420) aufweist
eine Längs-Mittellinie, die eine Längs-Richtung definiert, und eine Quer-Mittellinie, die eine Quer-Richtung definiert, wobei das Kleidungsstück umfasst:

   ein elastisches Taillenband (422);
   ein vorderes Feld (430) mit ersten und zweiten Abschnitten (436, 438), wobei der erste Abschnitt (436) einen größeren Dehnungs-Widerstand in der Quer-Richtung als der des zweiten Abschnittes (438) aufweist; ein hinteres Feld (440) mit ersten und zweiten Abschnitten (446, 448), wobei der erste Abschnitt einen größeren Dehnungs-Widerstand in der Quer-Richtung als der des zweiten Abschnittes aufweist;
   einen Schritt-Bereich (450), der zwischen dem vorderen Feld und dem hinteren Feld angeordnet ist und diese verbindet;
   ein Paar elastischer Bein-Öffnungen (460);
   ein Längsdehnungs-Steuerungselement (452), das entlang der Längs-Mittellinie in dem Schritt-Bereich angeordnet ist, wobei das Längsdehnungs-Steuerungselement (452) dazu dient, den Umfang der längsorientierten Dehnung des Schritt-Bereiches entlang der Längs-Mittellinie zu beschränken;
   ein vorderes Dehnungs-Steuerungselement (454), das in dem vorderen Feld (430) angeordnet ist und von dem Längsdehnungs-Steuerungselement (452) zu dem Taillenband verläuft, um die Kräfte von dem Längsdehnungs-Steuerungselement zu dem Taillenband zu lenken; und
   ein hinteres Dehnungs-Steuerungselement (456), das in dem hinteren Feld (440) angeordnet ist und von dem Längsdehnungs-Steuerungselement zu dem Taillenband (422) verläuft, um die Kräfte von dem Längsdehnungs-Steuerungselement zu dem Taillenband zu lenken.

3. Kleidungsstück (420) nach Anspruch 1, wobei der erste Abschnitt (436; 446) des vorderen oder hinteren Feldes einen größeren Dehnungs-Widerstand in der Längs-Richtung als der des zweiten Abschnittes (438; 448) des jeweiligen vorderen oder hinteren Feldes aufweist.

4. Kleidungsstück (420) nach Anspruch 1, wobei das Längsdehnungs-Steuerungselement (452) einen größeren Dehnungs-Widerstand in der Längs-Richtung als der des jeweiligen ersten Abschnittes (436; 446) des vorderen Feldes oder des hinteren Feldes aufweist.

5. Kleidungsstück (420) nach Anspruch 1, wobei die ersten und zweiten Abschnitte des vorderen oder hinteren Feldes einen Längsdehnungs-Modul aufweisen, wobei der Längsdehnungs-Modul des ersten Abschnittes (436; 446) größer als der Längsdehnungs-Modul des zweiten Abschnittes (438; 448) des vorderen oder hinteren Feldes ist.

6. Kleidungsstück (420) nach Anspruch 1, wobei die ersten und zweiten Abschnitte des vorderen oder hinteren Feldes einen Querdehnungs-Modul aufweisen, wobei der Querdehnungs-Modul des ersten Abschnittes (436; 446) größer als der Querdehnungs-Modul des zweiten Abschnittes (438; 448) des vorderen oder hinteren Feldes ist.

7. Kleidungsstück nach Anspruch 1, wobei das Kleidungsstück (420) ein Strick-Material aufweist.

8. Kleidungsstück (420) nach Anspruch 1, ferner umfassend ein Seitenfeld, welches das erste Feld mit dem hinteren Feld (440) verbindet.

9. Kleidungsstück (420) nach Anspruch 8, wobei das Seitenfeld einen größeren Dehnungs-Widerstand in der Quer-Richtung als der des zweiten Abschnittes des vorderen oder hinteren Feldes (440) aufweist.

23

**10.** Kleidungsstück (420) nach Anspruch 8, wobei der erste Abschnitt (436; 446) des vorderen oder hinteren Feldes einen größeren Dehnungs-Widerstand in der Quer-Richtung als der des Seitenfeldes aufweist.

**11.** Absorbierender Artikel nach Anspruch 1, ferner **dadurch gekennzeichnet, dass** das Mittel zum Halten des absorbierenden Kerns (10) in dem Schritt-Bereich in einer konvexen ausgebauchten Form ein Klebemittel oder mechanisches Befestigungsmittel ist, das außerhalb auf der Außenlage (16) des absorbierenden Elementes aufgebracht ist.

**12.** Absorbierender Artikel nach einem der Ansprüche 1 oder 2, ferner **dadurch gekennzeichnet, dass** das Mittel zum Halten des Kerns in dem Schritt-Bereich in einer konvexen ausgebauchten Form ein elastisch-zusammenziehbares Element mit mindestens einer Kraft-Komponente in CD-Richtung ist.

**13.** Absorbierender Artikel nach Anspruch 1, ferner **dadurch gekennzeichnet, dass** dieser eine Längen- und eine Breiten-Abmessung und eine zu diesen beiden Abmessungen senkrecht verlaufende z-Richtung aufweist,
ferner umfassend ein zweites Mittel, welches einstückig mit dem absorbierenden Element zum Halten des absorbierenden Kerns (10) in
der Urin-Aufnahmezone in einer konvexen ausgebauchten Form ist, welches ein elastisch-zusammenziehbares Element mit mindestens einer Kraft-Komponente in MD-Richtung ist, die mindestens eine zu
dem Träger während des Gebrauchs gerichtete z-gerichtete Kraft-Komponente ausübt.

**14.** Absorbierender Artikel nach einem der Ansprüche 1 bis 13, ferner **dadurch gekennzeichnet, dass** dieser ein oder mehrere konvex-konkave Umwandlungsmittel aufweist, das/die zwischen der Urin-Aufnahmezone und dem einen oder den zwei Taillen-Bereich/en des absorbierenden Elementes angeordnet ist/sind.

**15.** Absorbierender Artikel nach Anspruch 14, ferner **dadurch gekennzeichnet, dass** das konvex-konkave Umwandlungsmittel eine Gelenklinie ist oder Gelenklinien sind.

**16.** Absorbierender Artikel nach einem der Ansprüche 1 bis 15, femer **dadurch gekennzeichnet, dass** die Urin-Aufnahmezone ausgebildet ist, um die konvexe Ausbauchung des absorbierenden Elementes für mindestens eine Länge von 50 mm während der Verwendung und des Gebrauchs zu halten.

**17.** Absorbierender Artikel nach einem der Ansprüche 1 bis 16, ferner **dadurch gekennzeichnet, dass** der Artikel ferner ein Fixierungsmittel vom Typus Klebemittel umfasst, die lokal auf der Haut des Trägers zum Fixieren des Artikels an dem Körper des Trägers angebracht sind.

**18.** Absorbierender Artikel nach einem der vorherigen Ansprüche, ferner **dadurch gekennzeichnet, dass** ein Hohlraum im Gebrauch gebildet ist zwischen der Urin-Aufnahmezone mit einer konvexen Formgebung mit mindestens einer der konkav-geformten Taillenzonen und dem Körper des Trägers während des Gebrauchs.

**19.** Absorbierender Artikel nach Anspruch 1, wobei das absorbierende Element und das Kleidungsstück (420) durch ein oder mehrere Befestigungsmittel aneinander befestigt sind.

**20.** Absorbierender Artikel nach Anspruch 19, wobei mindestens eines der Befestigungsmittel eine Klebeverbindung zwischen den zwei Elementen zum Halten der konvexen Ausbauchung mindestens in der Urin-Aufnahmezone ist.

**21.** Absorbierender Artikel nach Anspruch 20, wobei mindestens eines der Befestigungsmittel ein lösbarer Klebstoff oder mechanisches Befestigungsmittel ist.

**22.** Absorbierender Artikel nach Anspruch 21, wobei mindestens eines der Befestigungsmittel entlang der Längs-Mittellinie der beiden Elemente angeordnet ist.


**Revendications**

**1.** Article absorbant comportant un élément absorbant, comprenant une zone de charge urinaire, et un vêtement (420) destiné à maintenir ledit élément absorbant, dans lequel ledit élément absorbant comprend une forme convexe protubérante de l'âme absorbante (10) orientée vers le haut en direction de l'utilisateur au moins pour des parties de ladite zone de charge urinaire,

et dans lequel ledit vêtement (420) comprend un élément de réglage d'étirement longitudinal, placé le long de la ligne médiane longitudinale de l'article au moins dans la zone de charge urinaire, en coïncidence avec la zone protubérante de forme convexe,

**caractérisé en ce que** ladite forme protubérante convexe, au moins de la zone de charge urinaire, est réalisée grâce à un pli de l'élément absorbant le long de la ligne médiane longitudinale de l'article au moins dans ladite zone de charge urinaire, de sorte qu'au moins des parties de la feuille de fond recouvrant l'âme absorbante soient en contact direct les unes avec les autres.

2. Article absorbant selon la revendication 1, dans lequel ledit vêtement (420) comporte :

une ligne médiane longitudinale, définissant une direction longitudinale, et une ligne médiane latérale, définissant une direction latérale, ledit vêtement comprenant :

une bande de ceinture élastifiée (422) ;
un panneau avant (430) présentant des première et deuxième parties (436, 438), ladite première partie (436) ayant une résistance à l'étirement dans la direction latérale supérieure à celle de ladite deuxième partie (438) ;
un panneau arrière (440) présentant des première et deuxième parties (446, 448), ladite première partie ayant une résistance à l'étirement dans la direction latérale supérieure à celle de ladite deuxième partie ;
une région d'entrejambe (450) placée entre ledit panneau avant et ledit panneau arrière et réunissant ces deux panneaux ;
une paire d'ouvertures de jambe élastifiées (460) ;
un élément (452) de réglage d'étirement longitudinal, placé le long de ladite ligne médiane longitudinale dans ladite région d'entrejambe, ledit élément (452) de réglage d'étirement longitudinal ayant la fonction de limiter l'étendue de l'étirement orienté longitudinalement de ladite région d'entrejambe le long de la ligne médiane longitudinale ;
un élément (454) de réglage d'étirement avant placé dans ledit panneau avant (430) et s'étendant dudit élément (452) de réglage d'étirement longitudinal à ladite bande de ceinture afin de diriger les forces dudit élément de réglage d'étirement longitudinal vers ladite bande de ceinture ; et
un élément (456) de réglage d'étirement arrière placé dans ledit panneau arrière (440) et s'étendant dudit élément de réglage d'étirement longitudinal vers ladite bande de ceinture (422) afin de diriger les forces dudit élément de réglage d'étirement longitudinal vers ladite bande de ceinture.

3. Vêtement (420) selon la revendication 1, dans lequel ladite première région (436 ; 446) dudit panneau avant ou arrière a une résistance à l'étirement dans la direction longitudinale supérieure à celle de ladite deuxième partie (438 ; 448) dudit panneau respectif avant ou arrière.

4. Vêtement (420) selon la revendication 1, dans lequel ledit élément (452) de réglage d'étirement longitudinal a une résistance à l'étirement dans la direction longitudinale supérieure à celle de la première partie (436 ; 446) respective dudit panneau avant ou dudit panneau arrière.

5. Vêtement (420) selon la revendication 1, dans lequel lesdites première et deuxième parties dudit panneau avant ou arrière ont un certain module d'étirage longitudinal, ledit module d'étirage longitudinal de ladite première partie (436 ; 446) étant supérieur audit module d'étirage longitudinal de ladite deuxième partie (438 ; 468) dudit panneau avant ou arrière.

6. Vêtement (420) selon la revendication 1, dans lequel lesdites première et deuxième parties dudit panneau avant ou arrière ont un certain module d'étirage latéral, ledit module d'étirage latéral de ladite première partie (436 ; 446) étant supérieur audit module d'étirage latéral de ladite deuxième partie (438 ; 468) dudit panneau avant ou arrière.

7. Vêtement selon la revendication 1, dans lequel ledit vêtement (420) comprend un matériau de tricot.

8. Vêtement (420) selon la revendication 1, comprenant, en outre, un panneau latéral réunissant ledit premier panneau audit panneau arrière (440).

9. Vêtement (420) selon la revendication 8, dans lequel ledit panneau latéral a une résistance à l'étirement dans la direction latérale supérieure à celle de ladite deuxième partie dudit panneau avant ou arrière (440).

**10.** Vêtement (420) selon la revendication 8, dans lequel ladite première partie (436 ; 446) dudit panneau avant ou arrière a une résistance à l'étirement dans la direction latérale supérieure à celle dudit panneau latéral.

**11.** Article absorbant selon la revendication 1, **caractérisé, en outre, en ce que** lesdits moyens destinés à maintenir l'âme absorbante (10) dans la région d'entrejambe dans une forme protubérante convexe est un moyen d'attache adhésif ou mécanique appliqué extérieurement sur la feuille de fond (16) de l'élément absorbant.

**12.** Article absorbant selon l'une quelconque des revendications 1 ou 2, **caractérisé, en outre, en ce que** ledit moyen destiné à maintenir l'âme dans la région d'entrejambe dans une forme protubérante convexe est un élément élastiquement contractile comportant au moins une composante de force de sens transversal à la machine.

**13.** Article absorbant selon la revendication 1, **caractérisé, en outre, en ce qu'**il a une dimension de longueur et une dimension de largeur, et une direction z perpendiculaire à ces deux dimensions,
comprenant, en outre, un deuxième moyen qui est unitaire avec l'élément absorbant pour maintenir l'âme absorbante (10) située dans la zone de charge urinaire dans une forme protubérante convexe, qui est un élément élastiquement contractile ayant au moins une composante de force de sens machine exerçant au moins une composante de force de direction z orientée en direction de l'utilisateur durant le port.

**14.** Article absorbant selon l'une quelconque des revendications 1 à 13, **caractérisé, en outre, en ce qu'**il comprend, en outre, un ou plusieurs moyen(s) de transition convexo-concave(s) placé(s) entre la zone de charge urinaire et l'une des régions de ceinture ou les deux régions de ceinture de l'élément absorbant.

**15.** Article absorbant selon la revendication 1, **caractérisé, en outre, en ce que** ledit ou lesdits moyen(s) de transition convexo-concave(s) est ou sont une ou des ligne(s) d'articulation.

**16.** Article absorbant selon l'une quelconque des revendications 1 à 15, **caractérisé, en outre, en ce que** la zone de charge urinaire est conçue pour maintenir ladite forme protubérante convexe de l'élément absorbant pour au moins une longueur de 50 mm durant l'application et durant l'utilisation.

**17.** Article absorbant selon l'une quelconque des revendications 1 à 16, **caractérisé, en outre, en ce que** l'article comprend, en outre, un moyen de fixation du type adhésif qui est appliqué localement sur la peau de l'utilisateur dans le but de fixer l'article sur le corps de l'utilisateur.

**18.** Article absorbant selon l'une quelconque des revendications précédentes, **caractérisé, en outre, en ce qu'**un vide est formé en utilisation entre la zone de charge urinaire ayant une conformation convexe avec au moins une des zones de ceinture de forme concave et le corps de l'utilisateur durant le port.

**19.** Article absorbant selon la revendication 1,
dans lequel ledit élément absorbant et ledit vêtement (420) sont fixés l'un sur l'autre grâce à un ou plusieurs moyen(s) de fixation.

**20.** Article absorbant selon la revendication 19, dans lequel au moins un desdits moyens de fixation est une liaison adhésive entre les deux éléments, destinée à maintenir la forme protubérante convexe au moins dans la zone de charge urinaire.

**21.** Article absorbant selon la revendication 20,
dans lequel au moins un desdits moyens de fixation est un adhésif libérable ou un moyen d'attache mécanique.

**22.** Article absorbant selon la revendication 19, dans lequel au moins un desdits moyens de fixation est placé le long de la ligne médiane longitudinale des deux éléments.

Fig. 1a

Fig. 1b

Fig. 1c

**Fig. 2a**

**Fig. 2b**

Fig. 3

Fig. 4

**Fig. 5**

Fig. 6

620

630

636

638

680

_Fig. 8_

620

640

630
636

680

646

638

648

_Fig. 7_

720

760

*Fig. 9*